(19) Europäisches Patentamt / European Patent Office / Office européen des brevets

(11) **EP 2 588 439 B1**

(12) **EUROPÄISCHE PATENTSCHRIFT**

(45) Veröffentlichungstag und Bekanntmachung des Hinweises auf die Patenterteilung:
**23.04.2014 Patentblatt 2014/17**

(21) Anmeldenummer: **11726423.4**

(22) Anmeldetag: **21.06.2011**

(51) Int Cl.:
***C07C 51/00*** (2006.01) ***C07C 53/02*** (2006.01)

(86) Internationale Anmeldenummer:
**PCT/EP2011/060269**

(87) Internationale Veröffentlichungsnummer:
**WO 2012/000823 (05.01.2012 Gazette 2012/01)**

(54) **VERFAHREN ZUR HERSTELLUNG VON AMEISENSÄURE DURCH UMSETZUNG VON KOHLENDIOXID MIT WASSERSTOFF**

PROCESS FOR THE PREPARATION OF FORMIC ACID BY REACTING CARBONDIOXID WIT HYDROGEN

PROCÉDÉ DE PRODUCTION D'ACIDE FORMIQUE PAR MISE EN RÉACTION DE DIOXYDE DE CARBONE AVEC DE L'HYDROGÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.06.2010 EP 10167705**

(43) Veröffentlichungstag der Anmeldung:
**08.05.2013 Patentblatt 2013/19**

(73) Patentinhaber: **BASF SE**
**67056 Ludwigshafen (DE)**

(72) Erfinder:
- **SCHAUB, Thomas**
  **67433 Neustadt (DE)**
- **FRIES, Donata Maria**
  **68199 Mannheim (DE)**
- **PACIELLO, Rocco**
  **67098 Bad Dürkheim (DE)**
- **MOHL, Klaus-Dieter**
  **68766 Hockenheim (DE)**
- **SCHÄFER, Martin**
  **67269 Grünstadt (DE)**
- **RITTINGER, Stefan**
  **68199 Mannheim (DE)**
- **SCHNEIDER, Daniel**
  **68159 Mannheim (DE)**

(56) Entgegenhaltungen:
**WO-A1-86/02066** **WO-A1-2008/116799**
**DE-T2- 68 920 933**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 588 439 B1

**Beschreibung**

Die Erfindung betrifft ein Verfahren zur Herstellung von Ameisensäure durch Umsetzung von Kohlendioxid mit Wasserstoff in einem Hydrierreaktor in Gegenwart eines Katalysators, enthaltend ein Element aus der 8, 9. oder 10. Gruppe des Periodensystems, eines tertiären Amins und eines polaren Lösungsmittels, unter Bildung von Ameisensäure/Amin-Addukten als Intermediaten, die anschließend thermisch gespalten werden.

Addukte aus Ameisensäure und tertiären Aminen können thermisch in freie Ameisensäure und tertiäres Amin gespalten werden und dienen daher als Intermediate in der Herstellung von Ameisensäure.

Ameisensäure ist ein wichtiges und vielseitig einsetzbares Produkt. Sie wird beispielsweise zum Ansäuern bei der Herstellung von Futtermitteln, als Konservierungsmittel, als Desinfektionsmittel, als Hilfsstoff in der Textil- und Lederindustrie, als Gemisch mit ihren Salzen zur Enteisung von Flugzeugen und Start- und Landebahnen sowie als Synthesebaustein in der chemischen Industrie verwendet.

Die genannten Addukte aus Ameisensäure und tertiären Aminen (DE68920933) können auf verschiedene Art und Weise hergestellt werden, beispielsweise (i) durch direkte Umsetzung des tertiären Amins mit Ameisensäure, (ii) durch Hydrolyse von Methylformiat zu Ameisensäure in Gegenwart des tertiären Amins oder (iii) durch katalytische Hydratisierung von Kohlenmonoxid oder Hydrierung von Kohlendioxid zu Ameisensäure in Gegenwart des tertiären Amins. Das letztgenannte Verfahren der katalytischen Hydrierung von Kohlendioxid hat den besonderen Vorteil, dass Kohlendioxid in großen Mengen verfügbar und hinsichtlich seiner Quelle flexibel ist.

Großtechnisch aussichtsreich erscheint dabei vor allem die katalytische Hydrierung von Kohlendioxid in Gegenwart von Aminen (W. Leitner, Angewandte Chemie 1995, 107, Seiten 2391 bis 2405; P. G. Jessop, T. Ikariya, R. Noyori, Chemical Reviews 1995, 95, Seiten 259 bis 272). Die hierbei gebildeten Addukte aus Ameisensäure und Aminen lassen sich thermisch in Ameisensäure und das eingesetzte, in die Hydrierung rückführbare Amin, spalten.

Der für die Reaktion notwendige Katalysator enthält ein oder mehrere Elements aus der 8., 9. oder 10. Gruppe des Periodensystems, also Fe, Co, Ni, Ru, Rh, Pd, Os, Ir und/oder Pt. Bevorzugt enthält der Katalysator Ru, Rh, Pd, Os, Ir und/oder Pt, besonders bevorzugt Ru, Rh und/oder Pd und ganz besonders bevorzugt Ru.

Um ein wirtschaftliches Verfahren zu ermöglichen, muss der verwendete Katalysator aus zwei Gründen möglichst vollständig aus dem Produktstrom abgetrennt und in die Reaktion zurückgeführt werden:

(1) große Verluste des teuren Katalysators würden erhebliche Zusatzkosten verursachen und wären für einen wirtschaftlichen Betrieb des Verfahrens prohibitiv.

(2) bei der thermischen Spaltung der Ameisensäure/Amin-Addukte sollte möglichst wenig Katalysator vorliegen, da dieser unter Abwesenheit eines $CO_2$- und/oder $H_2$-Druckes auch die Rückreaktion katalysiert und damit zu Verlusten an der gebildeten Ameisensäure führt.

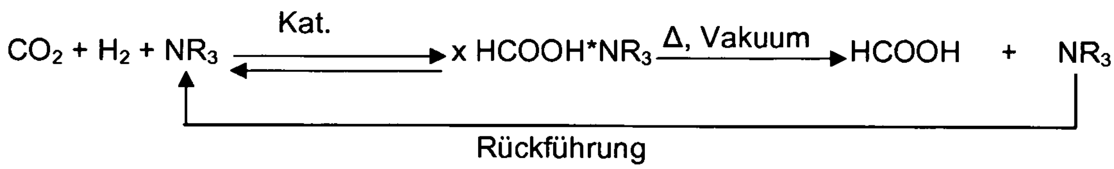


Bildung von Ameisensäure durch $CO_2$-Hydrierung (x = 0.4 - 3)

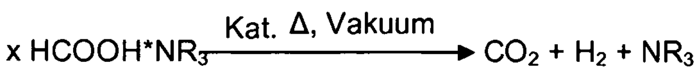


Zersetzung von Ameisensäure/Amin-Addukten unter Anwesenheit eines Katalysators (x = 0.4 - 3)

Die Übergangsmetall-katalysierte Zersetzung von Ameisensäure wurde vor allem in letzter Zeit ausführlich beschrieben: C. Fellay, N. Yan, P.J. Dyson, G. Laurenczy Chem. Eur. J. 2009, 15, 3752-3760; C. Fellay, P.J. Dyson, G. Laurenczy Angew. Chem. 2008, 120, 4030-4032; B. Loges, A. Boddien, H. Junge, M. Beller Angew. Chem. 2008, 120, 4026-4029; F. Joó ChemSusChem 2008, 1, 805-808; S. Enthaler ChemSusChem 2008, 1, 801-804; S. Fukuzumi, T. Kobayashi, T. Suenobu ChemSusChem 2008, 1, 827-834;A. Boddien, B. Loges, H. Junge, M. Beller ChemSusChem 2008, 1, 751-758.

Die hierbei eingesetzten Katalysatoren eignen sich grundsätzlich auch zur Hydrierung von $CO_2$ zu Ameisensäure (P.G. Jessop, T. Ikariya, R. Noyori Chem. Rev. 1995, 95, 259-272; P.G. Jessop, F. Joó, C.C. Tai Coord. Chem. Rev. 2004, 248, 2425-2442; P.G. Jessop, Homogeneous Hydrogenation of Carbon Dioxide, in: The Handbook of Homogeneous Hydrogenation, Hrsg.: J.G. de Vries, C.J. Elsevier, Volume 1, 2007, Wiley-VCH, S. 489-511). Somit müssen die Hydrierkatalysatoren vor der thermischen Spaltung abgetrennt werden, um die unerwünschte Ameisensäurezersetzung zu unterbinden.

WO 2008/116.799 offenbart ein Verfahren zur Hydrierung von Kohlendioxid in Gegenwart eines in Lösung suspendierten oder homogen gelösten Katalysators enthaltend ein Übergangsmetall der VIII. Nebengruppe (Gruppe 8, 9, 10), eines tertiären Amins mit mindestens einer Hydroxylgruppe und eines polaren Lösungsmittels zu einem Addukt aus Ameisensäure und dem tertiären Amin. Durch die Hydroxylgruppe(n) im tertiären Amin wird gegenüber dem sonst üblicherweise eingesetzten Triethylamin eine erhöhte Kohlendioxid-Löslichkeit erreicht. Als bevorzugte Homogenkatalysatoren sind $RuH_2L_4$ mit einzähnigen Phosphor-basierten Liganden L und $RuH_2(LL)_2$ mit zweizähnigen Phosphor-basierten Liganden LL und als besonders bevorzugt $RuH_2[P(C_6H_5)_3]_4$ genannt. Als polare Lösungsmittel sind Alkohole, Ether, Sulfolane, Dimethylsulfoxid und Amide, deren Siedepunkt bei Normaldruck mindestens 5°C oberhalb dem von Ameisensäure liegt, genannt. Auch die bevorzugt einzusetzenden tertiären Amine weisen einen Siedepunkt oberhalb dem der Ameisensäure auf. Da keine Phasentrennung stattfindet, erfolgt die Aufarbeitung des gesamten Reaktionsaustrags durch Destillation, gegebenenfalls nach vorheriger Abtrennung des Katalysators, wobei dabei das gebildete Addukt aus Ameisensäure und dem tertiären Amin thermisch gespalten und die freigesetzte Ameisensäure als Kopfprodukt gewonnen wird. Das Sumpfprodukt enthaltend tertiäres Amin, polares Lösungsmittel und gegebenenfalls Katalysator wird zur Hydrierstufe zurückgeführt.

Nachteilig an diesem Verfahren ist die Zuführung des gesamten flüssigen Reaktionsaustrags in die Vorrichtung zur thermischen Spaltung und Destillation, gegebenenfalls nach vorgeschalteter spezifischer Abtrennung des homogenen Katalysators durch einen separaten Verfahrensschritt wie beispielsweise einer Extraktions-, Adsorptions- oder Ultrafiltrationsstufe. Als Folge davon ist die Vorrichtung zur thermischen Spaltung und Destillation hinsichtlich der höheren Flüssigkeitsbelastung als auch hinsichtlich der spezifischeren Trenneigenschaften größer und komplexer auszulegen, was sich unter anderem auch in den Investitionskosten niederschlägt (zum Beispiel via Ingenieurleistung, Material, Flächenbedarf). Zudem bedingt die höhere Flüssigkeitsbelastung auch einen höheren Energiebedarf.

Die grundlegenden Arbeiten zur katalytische Hydrierung von Kohlendioxid zu Ameisensäure wurden jedoch bereits in den 1970er und 1980er Jahren gemacht. Als Ausfluss davon dürften wohl auch die in EP 0 095 321 A, EP 0 151 510 A und EP 0 181 078 A zum Patent angemeldeten Verfahren der BP Chemicals Ltd zu verstehen sein. Alle drei Schriften beschreiben die Hydrierung von Kohlendioxid in Gegenwart eines homogenen Katalysators enthaltend ein Übergangsmetall der VIII. Nebengruppe (Gruppe 8, 9, 10), eines tertiären Amins und eines polaren Lösungsmittels zu einem Addukt aus Ameisensäure und dem tertiären Amin. Als bevorzugte Homogenkatalysatoren sind in EP 0 095 321 A und EP 0 181 078 A jeweils Ruthenium-basierte Carbonyl-, Halogenid- und/oder Triphenylphosphin-haltige Komplexkatalysatoren und in EP 0 151 510 A Rhodium/Phosphin-Komplexe genannt. Bevorzugte tertiäre Amine sind $C_1$-$C_{10}$-Trialkylamine, insbesondere die kurzkettigen $C_1$-$C_4$-Trialkylamine, sowie cyclische und/oder verbrückte Amine wie 1,8-Diazabicyclo[5.4.0]undec-7-en, 1,4-Diazabicyclo[2.2.2]octan, Pyridin oder Picoline. Die Hydrierung erfolgt bei einem Kohlendioxid-Partialdruck von bis zu 6 MPa (60 bar), einem Wasserstoff-Partialdruck von bis zu 25 MPa (250 bar) und einer Temperatur von etwa Raumtemperatur bis 200°C.

EP 0 095 321 A und EP 0 151 510 A lehren den Einsatz eines Alkohols als polares Lösungsmittel. Da jedoch primäre Alkohole zur Bildung von Ameisensäureestern (organischen Formiaten) neigen, sind sekundäre Alkohole, insbesondere Isopropanol, bevorzugt. Zudem wird die Gegenwart von Wasser als vorteilhaft beschrieben. Gemäß den Beispielen aus EP 0 095 321 A erfolgt die Aufarbeitung des Reaktionsaustrags durch eine direkt nachfolgende zweistufige Destillation, bei der in der ersten Stufe die Leichtsieder Alkohol, Wasser, tertiäres Amin und in der zweiten Stufe unter Vakuumbedingungen das Addukt aus Ameisensäure und dem tertiären Amin über Kopf abgetrennt werden. EP 0 151 510 A lehrt ebenfalls eine destillative Aufarbeitung, jedoch unter Verweis auf EP 0 126 524 A mit nachfolgendem Austausch des tertiären Amins im destillativ abgetrennten Addukt vor dessen thermischer Spaltung durch eine schwächere, weniger flüchtige Stickstoffbase, um die nachfolgende thermische Spaltung zur Darstellung der freien Ameisensäure zu erleichtern, beziehungsweise erst möglich zu machen.

EP 0 181 078 A lehrt die gezielte Auswahl des polaren Lösungsmittels anhand von drei wesentlichen Kriterien, die gleichzeitig erfüllt sein müssen:

(i) der homogene Katalysator muss im polaren Lösungsmittel löslich sein;
(ii) das polare Lösungsmittel darf die Hydrierung nicht nachteilig beeinflussen; und
(iii) das gebildete Addukt aus Ameisensäure und dem tertiären Amin sollte leicht vom polaren Lösungsmittel abtrennbar sein.

Als besonders geeignete polare Lösungsmittel sind verschiedene Glykole und Phenylpropanole genannt.

Die Aufarbeitung des Reaktionsaustrags gemäß der Lehre von EP 0 181 078 A erfolgt derart, dass zunächst in einem Verdampfer die gasförmigen Komponenten (vor allem nicht umgesetzte Edukte Wasserstoff und Kohlendioxid) über Kopf und der homogene Katalysator gelöst im polaren Lösungsmittel über Sumpf abgetrennt und zur Hydrierstufe

zurückgeführt werden. Aus der verbleibenden Flüssigphase enthaltend das Addukt aus Ameisensäure und dem tertiären Amin, freies tertiäres Amin und gegebenenfalls Wasser wird dann anschließend das Addukt aus Ameisensäure und dem tertiären Amin abgetrennt und der verbleibende Teil der Flüssigphase enthaltend das freie tertiäre Amin und gegebenenfalls Wasser zur Hydrierstufe zurückgeführt. Die Abtrennung kann durch Destillation oder durch Phasentrennung des Zweiphasensystems (Dekantierung) erfolgen.

Eine weitere wesentliche Lehre aus EP 0 181 078 A ist der anschließende, zwingende Austausch des tertiären Amins im abgetrennten Addukt vor dessen thermischer Spaltung durch eine schwächere, weniger flüchtige Stickstoffbase, um die nachfolgende thermische Spaltung zur Darstellung der freien Ameisensäure zu erleichtern beziehungsweise erst möglich zu machen. Als besonders geeignete schwächere Stickstoffbasen sind Imidazolderivate wie beispielsweise 1-n-Butylimidazol genannt.

Nachteilig am Verfahren des EP 0 181 078 A ist die sehr aufwändige, vierstufige Aufarbeitung des Reaktionsaustrags durch

(i) Abtrennung der gasförmigen Komponenten sowie des homogenen Katalysators und des polaren Lösungsmittels in einem Verdampfer und Rückführung zur Hydrierstufe;
(ii) Abtrennung des Addukts aus Ameisensäure und dem tertiären Amin in einer Destillationskolonne oder einem Phasenscheider und Rückführung des verbleibenden Flüssigkeitsstroms zur Hydrierstufe;
(iii) Austausch des tertiären Amins im Addukt aus Ameisensäure und dem tertiären Amin durch eine schwächere, weniger flüchtige Stickstoffbase in einem Reaktionskessel mit aufgesetzter Destillationskolonne und Rückführung des freigesetzten tertiären Amins zur Hydrierstufe; und
(iv) thermische Spaltung des Addukts aus Ameisensäure und der schwächeren Stickstoffbase und Rückführung der freigesetzten schwächeren Stickstoffbase zur Basenaustauschstufe.

Ein weiterer, wesentlicher Nachteil am Verfahren des EP 0 181 078 A, sowie auch an den Verfahren der EP 0 095 321 A und EP 0 151 510 A ist die Tatsache, dass sich das Addukt aus Ameisensäure und dem tertiären Amin in Gegenwart des homogenen Katalysators bei der Aufarbeitung im Verdampfer zum Teil wieder in Kohlendioxid und Wasserstoff rückspaltet. In EP 0 329 337 A ist daher als Lösung des Problems die Zugabe eines Zersetzungsinhibitors vorgeschlagen, welcher den homogenen Katalysator reversibel inhibiert. Als bevorzugte Zersetzungsinhibitoren sind Kohlenmonoxid und Oxidationsmittel genannt. Nachteilig daran ist jedoch die Einführung weiterer Substanzen in das Gesamtverfahren und die Notwendigkeit, den inhibierten homogenen Katalysator vor dessen erneutem Einsatz wieder zu aktivieren.

Auch EP 0 357 243 A befasst sich mit dem Nachteil der teilweisen Rückspaltung des Addukts aus Ameisensäure und dem tertiären Amin im Verfahren des EP 0 181 078 A durch die gemeinsame Aufarbeitung des Reaktionsaustrags im Verdampfer. Das in EP 0 357 243 A vorgeschlagene Verfahren lehrt bei der katalytischen Hydrierung des Kohlendioxids zu einem Addukt aus Ameisensäure und tertiärem Amin den Einsatz eines homogenen Katalysators enthaltend ein Übergangsmetall der VIII. Nebengruppe (Gruppe 8, 9, 10), eines tertiären Amins und zweier unterschiedlicher Lösungsmittel, nämlich eines unpolaren und eines polaren, jeweils inerten Lösungsmittels, welche zwei nicht-mischbare flüssige Phasen bilden. Als unpolare Lösungsmittel sind aliphatische und aromatische Kohlenwasserstoffe, jedoch auch Phosphine mit aliphatischen und/oder aromatischen Kohlenwasserstoffresten genannt. Als polare Lösungsmittel sind Wasser, Glycerin, Alkohole, Polyole, Sulfolane oder deren Mischungen genannt, wobei Wasser bevorzugt ist. Im unpolaren Lösungsmittel löst sich der homogene Katalysator, im polaren Lösungsmittel das Addukt aus Ameisensäure und tertiärem Amin. Nach Beendigung der Reaktion werden beide Flüssigphasen separiert, beispielsweise durch Dekantierung, und die unpolare Phase enthaltend den homogenen Katalysator und das unpolare Lösungsmittel zur Hydrierstufe zurückgeführt. Die polare Phase enthaltend das Addukt aus Ameisensäure und tertiärem Amin und das polare Lösungsmittel wird dann einem zwingenden Austausch des tertiären Amins im Addukt vor dessen thermischer Spaltung durch eine schwächere, weniger flüchtige Stickstoffbase unterworfen, um die nachfolgende thermische Spaltung zur Darstellung der freien Ameisensäure zu erleichtern beziehungsweise erst möglich zu machen. Analog EP 0 181 078 A sind auch hier Imidazolderivate wie beispielsweise 1-n-Butylimidazol als besonders geeignete schwächere Stickstoffbasen genannt.

Nachteilig am Verfahren des EP 0 357 243 A ist die sehr aufwändige, dreistufige Aufarbeitung des Reaktionsaustrags durch

(i) Trennung der beiden Flüssigphasen und Rückführung der den homogenen Katalysator und das unpolare Lösungsmittel enthaltenden Phase zur Hydrierstufe;
(ii) Austausch des tertiären Amins im Addukt aus Ameisensäure und dem tertiären Amin der anderen Phase durch eine schwächere, weniger flüchtige Stickstoffbase in einem Reaktionskessel mit aufgesetzter Destillationskolonne und Rückführung des freigesetzten tertiären Amins zur Hydrierstufe; und
(iii) thermische Spaltung des Addukts aus Ameisensäure und der schwächeren Stickstoffbase und Rückführung

der freigesetzten schwächeren Stickstoffbase zur Basenaustauschstufe.

Ein weiterer Nachteil am Verfahren des EP 0 357 243 A ist der Einsatz zweier Lösungsmittel und somit die Einführung einer weiteren Substanz in das Gesamtverfahren.

Alternativ offenbart EP 0 357 243 A auch die Möglichkeit, nur ein Lösungsmittel einzusetzen. In diesem Fall entfällt die Zugabe des polaren Lösungsmittels, in dem sich ansonsten das Addukt aus Ameisensäure und dem tertiären Amin lösen würde. Das einzige hierbei eingesetzte Lösungsmittel ist das unpolare Lösungsmittel, welches den homogenen Katalysator löst. Auch bei dieser Alternative ist jedoch die sehr aufwändige, dreistufige Aufarbeitung wie oben beschrieben von Nachteil.

DE 44 31 233 A beschreibt ebenfalls die Hydrierung von Kohlendioxid in Gegenwart eines Katalysators enthaltend ein Übergangsmetall der VIII. Nebengruppe (Gruppe 8, 9, 10), eines tertiären Amins und eines polaren Lösungsmittels und Wasser zu einem Addukt aus Ameisensäure und dem tertiären Amin, bei dem jedoch der Katalysator heterogen vorliegt und die Aktivkomponente auf einem inerten Träger aufgebracht ist. Bevorzugte tertiäre Amine sind $C_1$-$C_8$-Trialkylamine, Polyamine mit 2 bis 5 Aminogruppen, aromatische Stickstoffheterocyclen wie Pyridin oder N-Methylimidazol, sowie cyclische und/oder verbrückte Amine wie N-Methylpiperidin, 1,8-Diazabicyclo[5.4.0]undec-7-en oder 1,4-Diazabicyclo[2.2.2]octan. Als geeignete polare Lösungsmittel sind die niedrig siedenden $C_1$-$C_4$-Monoalkohole genannt, wobei analog EP 0 095 321 A sekundäre Alkohole bevorzugt sind. Die Hydrierung erfolgt bei einem Gesamtdruck von 4 bis 20 MPa (40 bis 200 bar) und einer Temperatur von 50 bis 200°C. Zur Aufarbeitung des gebildeten Addukts aus Ameisensäure und tertiärem Amin lehrt DE 44 31 233 A den Einsatz bekannter Verfahren unter explizitem Verweis auf die in EP 0 357 243 A offenbarte Aufarbeitung unter Austausch des tertiären Amins im Addukt aus Ameisensäure und dem tertiären Amin durch eine schwächere, weniger flüchtige Stickstoffbase. Analog dem Verfahren nach EP 0 357 243 A ist auch beim Verfahren nach DE 44 31 233 A die sehr aufwändige, dreistufige Aufarbeitung des Reaktionsaustrags nachteilig.

Aufgabe der vorliegenden Erfindung war es, ein Verfahren zur Herstellung von Ameisensäure durch Hydrierung von Kohlendioxid zur Verfügung zu stellen, das die genannten Nachteile des Standes der Technik nicht oder nur in deutlich vermindertem Ausmaß aufweist und das zu konzentrierter Ameisensäure in hoher Ausbeute und hoher Reinheit führt. Ferner soll das Verfahren eine einfache oder zumindest einfachere Verfahrensführung erlauben, als im Stand der Technik beschrieben, insbesondere ein einfacheres Verfahrenskonzept zur Aufarbeitung des Austrags aus dem Hydrierreaktor, einfachere Verfahrensstufen, eine geringere Anzahl an Verfahrensstufen oder einfachere Apparate. Des Weiteren soll das Verfahren auch mit einem möglichst niedrigen Energiebedarf durchgeführt werden können. Insbesondere soll für das bislang nur unbefriedigend gelöste Problem der Rückführung des Katalysators eine effiziente Lösung angeboten werden unter gleichzeitiger Gewährleistung einer hohen Aktivität des Hydrierkatalysators.

Die Aufarbeitung des Austrags aus dem Hydrierreaktor soll insbesondere ausschließlich mit den im Verfahren bereits befindlichen Stoffen, ohne zusätzliche Hilfsstoffe, erfolgen, und diese sollen im Verfahren vollständig oder weitgehend vollständig recycliert werden.

Die Aufgabe wird gelöst durch ein Verfahren zur Herstellung von Ameisensäure durch Umsetzung von Kohlendioxid mit Wasserstoff in einem Hydrierreaktor in Gegenwart

- eines Katalysators, enthaltend ein Element aus der 8., 9. oder 10. Gruppe des Periodensystems,
- eines tertiären Amins, enthaltend mindestens 12 Kohlenstoffatome pro Molekül, sowie
- eines polaren Lösungsmittels, enthaltend einen oder mehrere Monoalkohole, ausgewählt aus Methanol, Ethanol, Propanolen und Butanolen,

unter Bildung von Ameisensäure/Amin-Addukten als Intermediaten, die anschließend thermisch gespalten werden, wobei ein tertiäres Amin eingesetzt wird, das einen um mindestens 5 °C höheren Siedepunkt als Ameisensäure aufweist, und wobei
sich bei der Umsetzung im Hydrierreaktor ein Reaktionsgemisch bildet, enthaltend das polare Lösungsmittel, die Ameisensäure/Amin-Addukte, das tertiäre Amin und den Katalysator, das aus dem Reaktor ausgetragen wird,
das dadurch gekennzeichnet ist, dass
die Aufarbeitung des Austrags aus dem Hydrierreaktor (I) nach den folgenden Verfahrensschritten durchgeführt wird:

1) Zugabe von Wasser zum Austrag aus dem Hydrierreaktor, wobei man zwei Flüssigphasen erhält,

2) Auftrennung der zwei Flüssigphasen in einem Phasentrenngefäß , Rückführung der Oberphase aus dem Phasentrenngefäß in den Hydrierreaktor und Weiterleitung der Unterphase aus dem Phasentrenngefäß in eine Extraktionsapparatur , worin

3) Reste an Katalysator mit demselben tertiären Amin, das in der Hydrierung eingesetzt wurde, extrahiert, das mit

Katalysator beladene tertiäre Amin in den Hydrierreaktor recycliert wird, und der katalysatorfreie Strom von mit den Ameisensäure/Amin-Addukten beladenem polaren Lösungsmittel in eine Destillationseinheit weitergeleitet wird, worin

4) eine fraktionierte Destillation durchgeführt wird unter Erhalt einer ersten Fraktion , die überwiegend das polare Lösungsmittel enthält, und die in den Hydrierreaktor recycliert wird, einer zweiten Fraktion , die überwiegend Wasser enthält, und die in den Austrag aus dem Hydrierreaktor recycliert oder verworfen wird, sowie einer Fraktion ,

5) in einem Phasentrenngefäß in eine Oberphase enthaltend überwiegend das tertiäre Amin und eine Unterphase, enthaltend überwiegend die Ameisensäure/Amin-Addukte, aufgetrennt wird, wobei

6) die Unterphase aus dem Phasentrenngefäß einer thermischen Spalteinheit zugeführt, und darin in einen das tertiäre Amin enthaltenden Strom , der in das Phasentrenngefäß zurückgeführt wird und in reine Ameisensäure gespalten wird, und wobei
ein das tertiäre Amin enthaltender Strom aus dem Phasentrenngefäß in die Extraktionsapparatur als selektives Lösungsmittel für den Katalysator geleitet wird.

In einer Ausführungsform wird in der Verfahrensstufe (6) lediglich ein Teilstrom des das tertiäre Amin enthaltenden Stroms aus dem Phasentrenngefäß in die Extraktionsapparatur als selektives Lösungsmittel für den Katalysator geleitet und der verbleibende Teil des das tertiäre Amin enthaltenden Stroms direkt in den Hydrierreaktor geleitet.

Der beim erfindungsgemäßen Verfahren bei der Hydrierung von Kohlendioxid einzusetzende Katalysator ist bevorzugt ein homogener Katalysator. Dieser enthält ein Element aus der 8., 9. oder 10. Gruppe des Periodensystems, also Fe, Co, Ni, Ru, Rh, Pd, Os, Ir und/oder Pt. Bevorzugt enthält der Katalysator Ru, Rh, Pd, Os, Ir und/oder Pt, besonders bevorzugt Ru, Rh und/oder Pd, und ganz besonders bevorzugt Ru.

Die genannten Elemente liegen in Form von komplexartigen Verbindungen im Reaktionsgemisch homogen gelöst vor. Der homogene Katalysator ist dabei so auszuwählen, dass dieser sich zusammen mit dem tertiären Amin in derselben Flüssigphase (B) anreichert. Unter "angereichert" ist dabei ein Verteilungskoeffizient des homogenen Katalysators

$$P = [\text{Konzentration homogener Katalysator in Flüssigphase (B)}] / [\text{Konzentration homogener Katalysator in Flüssigphase (A)}]$$

von > 1 zu verstehen. Die Auswahl des homogenen Katalysators erfolgt im Allgemeinen durch einen einfachen Versuch, indem der Verteilungskoeffizient des gewünschten homogenen Katalysators unter den geplanten Verfahrensbedingungen experimentell bestimmt wird.

Flüssigphase (A) ist hierbei die Unterphase aus dem Phasentrenngefäß in Verfahrensstufe 1).

Wegen ihrer guten Löslichkeit in tertiären Aminen verwendet man beim erfindungsgemäßen Verfahren als homogene Katalysatoren bevorzugt eine metallorganische Komplexverbindungen, die ein Element aus der 8., 9. oder 10. Gruppe des Periodensystems und mindestens eine Phosphin-Gruppe mit mindestens einem unverzweigten oder verzweigten, acyclischen oder cyclischen, aliphatischen Rest mit 1 bis 12 Kohlenstoffatomen enthalten, wobei einzelne Kohlenstoffatome auch durch >P- substituiert sein können. Im Sinne von verzweigten cyclischen aliphatischen Resten sind damit eingeschlossen auch Reste wie beispielsweise $-CH_2-C_6H_{11}$. Als geeignete Reste sind beispielsweise genannt Methyl, Ethyl, 1-Propyl, 2-Propyl, 1-Butyl, 2-Butyl, 1-(2-Methyl)propyl, 2-(2-Methyl)propyl, 1-Pentyl, 1-Hexyl, 1-Heptyl, 1-Octyl, 1-Nonyl, 1-Decyl, 1-Undecyl, 1-Dodecyl, Cyclopentyl, Cyclohexyl, Cycloheptyl und Cyclooctyl, Methylcyclopentyl, Methylcyclohexyl, 1-(2-Methyl)-pentyl 1-(2-Ethyl)-hexyl, 1-(2-Propyl)heptyl und Norbonyl. Bevorzugt enthält der unverzweigte oder verzweigte, acyclische oder cyclische, aliphatische Rest mindestens 1 sowie bevorzugt maximal 10 Kohlenstoffatome. Im Falle eines ausschließlich cyclischen Restes im oben genannten Sinne beträgt die Zahl der Kohlenstoffatome 3 bis 12 und bevorzugt mindestens 4 sowie bevorzugt maximal 8 Kohlenstoffatome. Bevorzugte Reste sind Ethyl, 1-Butyl, sec-Butyl, 1-Octyl und Cyclohexyl.

Die Phosphin-Gruppe kann einen, zwei oder drei der oben genannten unverzweigten oder verzweigten, acyclischen oder cyclischen, aliphatischen Reste enthalten. Diese können gleich oder verschieden sein. Bevorzugt enthält die Phosphin-Gruppe drei der oben genannten unverzweigten oder verzweigten, acyclischen oder cyclischen, aliphatischen Reste, wobei besonders bevorzugt alle drei Reste gleich sind. Bevorzugte Phosphine sind $P(n\text{-}C_nH_{2n+})_3$ mit n gleich 1 bis 10, besonders bevorzugt Tri-n-butylphosphin, Tri-n-octylphosphin und 1,2-Bis(dicyclohexylphosphino)ethan.

Wie bereits weiter oben erwähnt, können in den genannten unverzweigten oder verzweigten, acyclischen oder cyclischen, aliphatischen Resten einzelne Kohlenstoffatome auch durch >P- substituiert sein. Somit sind auch mehrzähnige, beispielsweise zwei- oder dreizähnige, Phosphin-Liganden mit umfasst. Diese enthalten bevorzugt die Gruppierung >P-$CH_2CH_2$-P< beziehungsweise

$$>P\text{-}CH_2CH_2\text{-}\overset{|}{P}\text{-}CH_2CH_2\text{-}P< .$$

Enthält die Phosphin-Gruppe noch andere Reste als die oben genannten unverzweigten oder verzweigten, acyclischen oder cyclischen, aliphatischen Reste, so entsprechen diese im Allgemeinen denen, die ansonsten üblicherweise bei Phosphin-Liganden für metallorganische Komplexkatalysatoren eingesetzt werden. Als Beispiele seien genannt Phenyl, Tolyl und Xylyl.

Die metallorganische Komplexverbindung kann eine oder mehrere, beispielsweise zwei, drei oder vier, der oben genannten Phosphin-Gruppen mit mindestens einem unverzweigten oder verzweigten, acyclischen oder cyclischen, aliphatischen Rest enthalten. Die restlichen Liganden des metallorganischen Komplexes können verschiedener Natur sein. Als exemplarische Beispiele seien genannt Hydrid, Fluorid, Chlorid, Bromid, Iodid, Formiat, Acetat, Propionat, Carboxylat, Acetylacetonat, Carbonyl, DMSO, Hydroxid, Trialkylamin, Alkoxid.

Die homogenen Katalysatoren können sowohl direkt in ihrer aktiven Form als auch ausgehend von üblichen Standardkomplexen wie beispielsweise [M(p-Cymene)$Cl_2$]$_2$, [M(benzol)$Cl_2$]$_n$, [M(COD)(allyl)], [$MCl_3$ x $H_2O$], [M(acetylacetonat)$_3$], [M$(DMSO)_4Cl_2$] mit M gleich Element aus der 8., 9. oder 10. Gruppe des Periodensystems unter Zugabe des beziehungsweise der entsprechenden Phosphin-Liganden erst unter Reaktionsbedingungen erzeugt werden.

Beim erfindungsgemäßen Verfahren bevorzugte homogene Katalysatoren sind [Ru$(P^nBu_3)_4(H)_2$], [Ru$(P^nOctyl_3)_4(H)_2$], [Ru$(P^nBu_3)_2$(1,2-bis(dicyclohexylphosphino)-ethan)$(H)_2$], [Ru$(P^nOctyl_3)_2$(1,2-bis(dicyclohexylphosphino)ethan)$(H)_2$], [Ru$(PEt_3)_4(H)_2$. Mit diesen können in der Hydrierung von Kohlendioxid TOF-Werte (turn-overfrequency) von größer 1000 $h^{-1}$ erzielt werden.

Beim Einsatz von homogenen Katalysatoren beträgt die eingesetzte Menge der genannten Metallkomponente im metallorganischen Komplex im Allgemeinen 0,1 bis 5000 Gew.-ppm, bevorzugt 1 bis 800 Gew.-ppm und besonders bevorzugt 5 bis 800 Gew.-ppm, jeweils bezogen auf das gesamte flüssige Reaktionsgemisch im Hydrierreaktor.

Der Verteilungskoeffizient des homogenen Katalysators bezogen auf die Menge an Ruthenium in der Aminphase und der das Ameisensäure/Amin-Addukt enthaltenden Produktphase ist nach der Hydrierung im Bereich von P größer 0,5, bevorzugtermaßen größer 1,0 und besonders bevorzugtermaßen größer 4.

Das beim erfindungsgemäßen Verfahren bei der Hydrierung von Kohlendioxid einzusetzende tertiäre Amin weist einen um mindestens 5°C höheren Siedepunkt als Ameisensäure auf. Dabei ist, wie üblich, bei der Angabe der relativen Lage von Siedepunkten von Verbindungen zueinander auf jeweils denselben Druck abzustellen. Das tertiäre Amin ist dabei so ausgewählt und mit dem polaren Lösungsmittel abgestimmt, dass das tertiäre Amin in der Oberphase im Hydrierreaktor angereichert vorliegt. Unter "angereichert" ist dabei ein Gewichtsanteil von > 50% des freien, das heißt nicht in Form des Ameisensäure/Amin-Addukts gebundenen, tertiären Amins in der Oberphase bezogen auf die Gesamtmenge an freiem, tertiärem Amins in beiden Flüssigphasen zu verstehen. Bevorzugt liegt der Gewichtsanteil bei > 90 %. Die Auswahl des tertiären Amins erfolgt im Allgemeinen durch einen einfachen Versuch, in dem die Löslichkeit des gewünschten tertiären Amins in beiden Flüssigphasen unter den geplanten Verfahrensbedingungen experimentell bestimmt wird. Die Oberphase kann zudem noch Teile des polaren Lösungsmittels sowie eines unpolaren inerten Lösungsmittel enthalten.

Bevorzugt weist das einzusetzende tertiäre Amin einen um mindestens 10°C, besonders bevorzugt einem um mindestens 50°C und ganz besonders bevorzugt einem um mindestens 100°C höheren Siedepunkt als Ameisensäure auf. Eine Einschränkung hinsichtlich eines oberen Grenzwertes für den Siedepunkt ist nicht erforderlich, da für das erfindungsgemäße Verfahren ein möglichst niedriger Dampfdruck des tertiären Amin grundsätzlich von Vorteil ist. Im Allgemeinen liegt der Siedepunkt des tertiären Amins bei einem, gegebenenfalls nach bekannten Methoden vom Vakuum auf 1013 hPa abs hochgerechneten Druck unterhalb von 500°C.

Das beim erfindungsgemäßen Verfahren bevorzugt einzusetzende tertiäre Amin ist ein Amin, enthaltend mindestens 12 Kohlenstoffatome pro Molekül der allgemeinen Formel (Ia)

$$NR^1R^2R^3 \qquad \text{(Ia)},$$

in der die Reste $R^1$ bis $R^3$ gleich oder verschieden sind und unabhängig voneinander einen unverzweigten oder verzweigten, acyclischen oder cyclischen, aliphatischen, araliphatischen oder aromatischen Rest mit jeweils 1 bis 16 Kohlenstoffatomen, bevorzugt 1 bis 12 Kohlenstoffatomen, darstellen, wobei einzelne Kohlenstoffatome unabhängig voneinander auch durch eine Heterogruppe ausgewählt aus der Gruppe -O- und >N- substituiert sein können sowie zwei oder alle drei Reste unter Bildung einer mindestens jeweils vier Atome umfassenden Kette auch miteinander verbunden sein können.

Als geeignete Amine seien beispielsweise genannt:

- Tri-n-butylamin, Tri-n-pentylamin, Tri-n-hexylamin, Tri-n-heptylamin, Tri-n-octylamin, Tri-n-nonylamin, Tri-n-decylamin, Tri-n-undecylamin, Tri-n-dodecylamin, Tri-n-tridecylamin, Tri-n-tetradecylamin, Tri-n-pentadecylamin, Tri-n-hexadecylamin, Tri-(2-ethylhexyl)amin.
- Dimethyldecylamin, Dimethyldodecylamin, Dimethyltetradecylamin, Ethyl-di-(2-propyl)amin ($Sdp_{1013\ hPa}$ = 127°C), Dioctyl-methylamin, Dihexylmethylamin.
- Tricyclopentylamin, Tricyclohexylamin, Tricycloheptylamin, Tricyclooctylamin und deren durch eine oder mehrere Methyl-, Ethyl-, 1-Propy-, 2-Propyl-, 1-Butyl-, 2-Butyl- oder 2-Methyl-2-propyl-Gruppen substituierte Derivate.
- Dimethyl-cyclohexylamin, Methyl-dicyclohexylamin, Diethyl-cyclohexylamin, Ethyl-dicyclohexylamin, Dimethyl-cyclopentylamin, Methyl-dicyclopentylamin.
- Triphenylamin, Methyl-diphenylamin, Ethyl-diphenylamin, Propyl-diphenylamin, Butyl-diphenylamin, 2-Ethyl-hexyl-diphenylamin, Dimethyl-phenylamin, Diethylphenylamin, Dipropyl-phenylamin, Dibutyl-phenylamin, Bis(2-Ethyl-hexyl)-phenylamin, Tribenzylamin, Methyl-dibenzylamin, Ethyl-dibenzylamin und deren durch eine oder mehrere Methyl-, Ethyl-, 1-Propy-, 2-Propyl-, 1-Butyl-, 2-Butyl- oder 2-Methyl-2-propyl-Gruppen substituierte Derivate.
- N-$C_1$-bis-$C_{12}$-Alkyl-piperidine, N,N-Di-$C_1$-bis-$C_{12}$-Alkyl-piperazine, N-$C_1$-bis-$C_{12}$-Alkyl-pyrrolidine, N-$C_1$-bis-$C_{12}$-Alkyl-imidazole und deren durch eine oder mehrere Methyl- , Ethyl-, 1-Propyl-, 2-Propyl-, 1-Butyl-, 2-Butyl- oder 2-Methyl-2-propyl-Gruppen substituierte Derivate.
- 1,8-Diazabicyclo[5.4.0]undec-7-en ("DBU"), 1,4-Diazabicyclo[2.2.2]octan ("DABCO"), N-Methyl-8-azabicyclo[3.2.1]octan ("Tropan"), N-Methyl-9-azabicyclo[3.3.1]nonan ("Granatan"), 1-Azabicyclo[2.2.2]octan ("Chinuclidin").

Beim erfindungsgemäßen Verfahren können natürlich auch Gemische einer beliebigen Anzahl verschiedener tertiärer Amine eingesetzt werden.

Besonders bevorzugt setzt man beim erfindungsgemäßen Verfahren als tertiäres Amin ein Amin der allgemeinen Formel (Ia), in der die Reste $R^1$ bis $R^3$ unabhängig voneinander ausgewählt sind aus der Gruppe $C_1$- bis $C_{12}$-Alkyl, $C_5$- bis $C_8$-Cycloalkyl, Benzyl und Phenyl, ein.

Besonders bevorzugt setzt man beim erfindungsgemäßen Verfahren als tertiäres Amin ein gesättigtes Amin, d.h. nur Einfachbindungen enthaltend, der allgemeinen Formel (Ia) ein.

Ganz besonders bevorzugt setzt man beim erfindungsgemäßen Verfahren als tertiäres Amin ein Amin der allgemeinen Formel (Ia) ein, in der die Reste $R^1$ bis $R^3$ unabhängig voneinander ausgewählt sind aus der Gruppe $C_5$- bis $C_8$-Alkyl, insbesondere Tri-n-pentylamin, Tri-n-hexylamin, Tri-n-heptylamin, Tri-n-octylamin, Dimethylcyclohexylamin, Methyldicyclohexylamin, Dioctyl-methylamin und Dimethyldecylamin.

Insbesondere setzt man als tertiäres Amin ein Amin der allgemeinen Formel (Ia) ein, in der die Reste $R^1$ bis $R^3$ unabhängig voneinander ausgewählt sind aus $C_5$- und $C_6$-Alkyl.

Bevorzugt liegt das tertiäre Amin beim erfindungsgemäßen Verfahren in allen Verfahrensstufen flüssig vor. Dies ist jedoch kein zwingendes Erfordernis. Es würde auch ausreichen, wenn das tertiäre Amin zumindest in geeigneten Lösungsmitteln gelöst wäre. Geeignete Lösungsmittel sind prinzipiell solche, welche hinsichtlich der Hydrierung von Kohlendioxid sowie der thermischen Spaltung des Addukts chemisch inert sind, in denen sich das tertiäre Amin und, im Falle des Einsatzes eines homogenen Katalysators, auch diesen gut lösen, umgekehrt polares Lösungsmittel sowie das Ameisensäure/Amin-Addukte schlecht lösen. In Frage kommen daher prinzipiell chemisch inerte, unpolare Lösungsmittel wie etwa aliphatische, aromatische oder araliphatische Kohlenwasserstoffe, beispielsweise Octan und höhere Alkane, Toluol, Xylole.

Das beim erfindungsgemäßen Verfahren bei der Hydrierung von Kohlendioxid einzusetzende polare Lösungsmittel weist einen um mindestens 5°C niedrigeren Siedepunkt als die zur Spaltung der Ameisensäure/Amin-Addukte bei gleichem Druck benötigte Temperatur auf. Das polare Lösungsmittel ist dabei so auszuwählen beziehungsweise mit dem tertiären Amin abzustimmen, dass das polare Lösungsmittel in der Unterphase angereichert vorliegt. Unter "angereichert" ist dabei ein Gewichtsanteil von > 50% des polaren Lösungsmittels in der Unterphase bezogen auf die Gesamtmenge an polarem Lösungsmittel in beiden Flüssigphasen zu verstehen. Bevorzugt liegt der Gewichtsanteil bei > 70%. Die Auswahl des polaren Lösungsmittels erfolgt im Allgemeinen durch einen einfachen Versuch, in dem die Löslichkeit des gewünschten polaren Lösungsmittels in beiden Flüssigphasen unter den geplanten Verfahrensbedingungen experimentell bestimmt wird.

Bei dem polaren Lösungsmittel kann es sich um ein reines polares Lösungsmittel als auch um ein Gemisch verschiedener polarer Lösungsmittel handeln, solange die an das Lösungsmittel gestellten oben genannten Bedingungen bezüglich Siedepunkt und Phasenverhalten zutreffen.

Bevorzugt weist das einzusetzende polare Lösungsmittel einen um mindestens 10°C, besonders bevorzugt einen um mindestens 50°C niedrigeren Siedepunkt als die zur Spaltung der Ameisensäure/Amin-Addukte bei gleichem Druck benötigte Temperatur. Bei Lösungsmittelgemischen liegen die Siedpunkte des eingesetzten Lösungsmittelgemisches bzw. eines Azeotrops oder Heteroazeotrops um mindestens 10°C, besonders bevorzugt einem um mindestens 50°C niedriger als die zur Spaltung der Ameisensäure/Amin-Addukte bei gleichem Druck benötigte Temperatur.

Als Stoftklassen, die als polare Lösungsmittel geeignet sind, kommen bevorzugt Alkohole sowie deren Ameisensäureester und Wasser in Frage. Die Alkohole weisen einen um mindestens 10°C, besonders bevorzugt einem um mindestens 50°C niedrigeren Siedepunkt als die zur Spaltung der Ameisensäure/Amin-Addukte bei gleichem Druck benötigte Temperatur auf, um die Veresterung der Alkohole mit Ameisensäure möglichst gering zu halten.

Als geeignete Alkohole sind Alkohole genannt, in welchen sich im Gemisch mit Wasser die Trialkylammoniumformiate bevorzugt lösen und diese Produktphase eine Mischungslücke mit dem freien Trialkylamin aufweist. Als geeignete Alkohole seien beispielsweise Methanol, Ethanol, 2-Methoxyethanol, 1-Propanol, 2-Propanol, 1-Butanol, 2-Butano, 2-Methyl-1-propanol genannt. Das Verhältnis von Alkohol zu Wasser ist so zu wählen, das sich zusammen mit dem Trialkylammoniumformiates und dem Trialkylamin ein zweiphasiges Gemisch bildet, in welchem sich der Großteil des Trialkylammoniumformiates, das Wasser sowie das polare Lösungsmittel in der Unterphase befindet, was im Allgemeinen durch einen einfachen Versuch bestimmt wird, in dem die Löslichkeit des gewünschten polaren Lösungsmittelgemisches in beiden Flüssigphasen unter den geplanten Verfahrensbedingungen experimentell bestimmt wird.

Das Molverhältnis des beim erfindungsgemäßen Verfahren einzusetzenden polaren Lösungsmittels oder Lösungsmittelgemisches zum eingesetzten tertiären Amin beträgt im Allgemeinen 0,5 bis 30 und bevorzugt 1 bis 20.

Erfindungsgemäß wird die Aufarbeitung des Austrags aus dem Hydrierreaktor in der Weise durchgeführt, dass in einem ersten Verfahrensschritt zum Austrag aus dem Hydrierreaktor Wasser zugegeben wird.

Die Zugabe von Wasser nach erfolgter Hydrierung hat die Auswirkung, dass sich die Verteilungskoeffizienten des Katalysators durch die Wasserzugabe zugunsten einer Anreicherung desselben in der Amin-Phase verbessern und dadurch eine effiziente Rückführung des Katalysator ermöglicht wird, ohne dessen Hydrieraktivität zu erniedrigen. Der Hydrieraustrag kann, je nach verwendetem polarem Lösungsmittel und Konzentration an Ameisensäure-Amin-Addukten zweiphasig sein, wobei sich dann durch Zugabe von Wasser die Ameisensäure-Amin-Addukte in der Produktphase (der Unterphase) anreichern. Im Falle von einphasigen Hydrierausträgen unter Verwendung von erfindungsgemäßen polaren Lösungsmitteln wird der Phasenzerfall unter Bildung der Produktphase (der Unterphase) sowie der Aminphase (der Oberphase) durch die Wasserzugabe induziert. Es wird bevorzugt Wasser zugegeben, bis ein Wassergehalt in der Produktphase von 0,1 bis 50 Gew.-%, bezogen auf das Gesamtgewicht der Unterphase aus dem Phasentrenngefäß II, erreicht ist, besonders bevorzugt 2 bis 30 Gew.- %, bezogen auf das Gesamtgewicht der Unterphase aus dem Phasentrenngefäß II. Das zuzuführende Wasser kann aus der Destillationseinheit IV stammen, in welcher der größte Teil des polaren Lösungsmittels sowie das Wasser vom Produktstrom (7) abgetrennt wird und/oder es kann sich auch um Wasser handeln, welches dem Prozess frisch zugeführt wird. Das Wasser kann sowohl nach der Entspannung des Austrages aus dem Hydrierreaktor auf Normaldruck, als auch vor der Entspannung desselben zugegeben werden.

Das bei der Hydrierung von Kohlendioxid einzusetzende Kohlendioxid kann fest, flüssig oder gasförmig eingesetzt werden. Es ist auch möglich, großtechnisch verfügbare, Kohlendioxid enthaltende Gasgemische zu verwenden, sofern diese weitgehend frei von Kohlenmonoxid, d.h. einen Volumenanteil von <1 % CO, sind. Der bei der Hydrierung von Kohlendioxid einzusetzende Wasserstoff ist im Allgemeinen gasförmig. Kohlendioxid und Wasserstoff können auch inerte Gase, wie etwa Stickstoff oder Edelgase, enthalten. Vorteilhafterweise liegt jedoch deren Gehalt unterhalb von 10 mol-% bezogen auf die Gesamtmenge an Kohlendioxid und Wasserstoff im Hydrierreaktor. Größere Mengen sind zwar gegebenenfalls ebenfalls noch tolerierbar, bedingen aber im Allgemeinen die Anwendung eines höheren Druckes im Reaktor, wobei dadurch weitere Kompressionsenergie erforderlich ist und der apparative Aufwand steigt.

Die Hydrierung von Kohlendioxid erfolgt in der Flüssigphase bevorzugt bei einer Temperatur von 20 bis 200°C und einem Gesamtdruck von 0,2 bis 30 MPa abs. Bevorzugt beträgt die Temperatur mindestens 30°C und besonders bevorzugt mindestens 40°C sowie bevorzugt höchstens 150°C, besonders bevorzugt höchstens 120°C und ganz besonders bevorzugt höchstens 80°C. Der Gesamtdruck beträgt bevorzugt mindestens 1 MPa abs und besonders bevorzugt mindestens 5 MPa abs sowie bevorzugt höchstens 15 MPa abs.

Der Partialdruck des Kohlendioxids beträgt dabei im Allgemeinen mindestens 0,5 MPa und bevorzugt mindestens 2 MPa sowie im Allgemeinen höchstens 8 MPa. Der Partialdruck des Wasserstoffs beträgt im Allgemeinen mindestens 0,5 MPa und bevorzugt mindestens 1 MPa sowie im Allgemeinen höchstens 25 MPa und bevorzugt höchstens 10 MPa.

Das Molverhältnis von Wasserstoff zu Kohlendioxid im Zulauf des Hydrierreaktors beträgt bevorzugt 0,1 bis 10 und besonders bevorzugt 1 bis 3.

Das Molverhältnis von Kohlendioxid zu tertiärem Amin im Zulauf des Hydrierreaktors beträgt im Allgemeinen

0,1 bis 10 und bevorzugt 0,5 bis 3.

Als Hydrierreaktoren können prinzipiell alle Reaktoren eingesetzt werden, welche für gas/flüssig-Reaktionen unter der gegebenen Temperatur und dem gegebenen Drucks grundsätzlich geeignet sind. Geeignete Standardreaktoren für flüssig-flüssig Reaktionssyteme sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt Rührkesselreaktoren, Rohrreaktoren oder Blasensäulenreaktoren.

Die Hydrierung von Kohlendioxid kann beim erfindungsgemäßen Verfahren diskontinuierlich oder kontinuierlich durchgeführt werden. Bei der diskontinuierlichen Fahrweise wird der Reaktor mit den gewünschten flüssigen und gegebenenfalls festen Einsatz- und Hilfsstoffen bestückt und anschließend Kohlendioxid und Wasserstoff auf den gewünschten Druck bei der gewünschten Temperatur aufgepresst. Nach Ende der Reaktion wird der Reaktor im Regelfall entspannt und die beiden gebildeten Flüssigphasen voneinander getrennt. Bei der kontinuierlichen Fahrweise werden die Einsatz- und Hilfsstoffe, einschließlich des Kohlendioxids und Wasserstoffs kontinuierlich zugegeben. Entsprechend wird die Flüssigphase kontinuierlich aus dem Reaktor abgeführt, so dass der Flüssigkeitsstand im Reaktor im Mittel gleich bleibt. Bevorzugt ist die kontinuierliche Hydrierung von Kohlendioxid.

Die mittlere Verweilzeit im Hydrierreaktor beträgt im Allgemeinen 10 Minuten bis 5 Stunden.

Die bei der Hydrierung von Kohlendioxid in Gegenwart des einzusetzenden Katalysators, des polaren Lösungsmittels und des tertiären Amins gebildeten Ameisensäure/Amin-Addukte weisen im Allgemeinen die allgemeine Formel (IIa)

$$NR^1R^2R^3 \cdot x_i\ HCOOH \qquad (IIa),$$

auf, in der die Reste $R^1$ bis $R^3$ den beim tertiären Amin (Ia) beschriebenen Resten entsprechen und $x_1$ gleich 0.4 bis 5, bevorzugt 0.7 bis 1.6 beträgt. Die jeweilige gemittelte Zusammensetzunge des Amin-Ameisensäureverhältnisses in den Produktphasen bei den jeweiligen Verfahrensschritten, d.h. der Faktor $x_i$, lassen sich beispielsweise durch Bestimmung des Ameisensäuregehaltes durch Titration mit einer alkoholischen KOH-Lösung gegen Phenolphthalein und des Amingehaltes durch Gaschromatographie bestimmen. Die Zusammensetzung der Ameisensäure/Amin-Addukte, d.h. der Faktor $x_i$ kann sich während der verschiedenen Verfahrensschritte ändern. So bilden sich zum Beispiel im Allgemeinen nach der Entfernung des polaren Lösungsmittel Addukte mit einem höheren Ameisensäuregehalt mit $x_2 > x_1$ mit $x_2$ 1 bis 4 aus, wobei das überschüssige, freie Amin eine Zweitphase ausbilden kann.

Bei der Hydrierung von Kohlendioxid nach dem erfindungsgemäßen Verfahren werden zwei Flüssigphasen gebildet. Die Unterphase ist mit den Ameisensäure/Amin-Addukten sowie dem polaren Lösungsmittel angereichert. Hinsichtlich der Ameisensäure/Amin-Addukte ist unter "angereichert" ein Verteilungskoeffizient der Ameisensäure/Amin-Addukte

P = [Konzentration Ameisensäure/Amin-Addukt (II) in Flüssigphase (A)] / [Konzentration Ameisensäure/Amin-Addukt (II) in Flüssigphase (B)]

von > 1 zu verstehen. Bevorzugt liegt der Verteilungskoeffizient bei ≥ 2 und besonders bevorzugt bei ≥ 5. Die Oberphase ist mit dem tertiären Amin angereichert. Im Falle des Einsatzes eines homogenen Katalysators liegt dieser ebenfalls in der Oberphase angereichert vor.

Die Flüssigphasen (A) und (B) haben dabei die bereits vorstehend definierte Bedeutung.

Die beiden gebildeten Flüssigphasen werden beim erfindungsgemäßen Verfahren voneinander getrennt und die Oberphase zum Hydrierreaktor rückgeführt. Gegebenenfalls ist auch eine Rückführung einer über beiden Flüssigphasen vorhandenen weiteren Flüssigphase enthaltend nicht umgesetztes Kohlendioxid sowie einer Gasphase enthaltend nicht umgesetztes Kohlendioxid und/oder nicht umgesetzten Wasserstoff zum Hydrierreaktor vorteilhaft. Gegebenenfalls ist es beispielsweise zur Ausschleusung von unerwünschten Nebenprodukten oder Verunreinigungen wünschenswert, einen Teil der Oberphase und/oder einen Teil der Kohlendioxid oder Kohlendioxid und Wasser- stoff enthaltenden flüssigen beziehungsweise gasförmigen Phasen aus dem Verfahren auszuschleusen.

Die Trennung der beiden Flüssigphasen erfolgt im Allgemeinen durch gravimetrische Phasentrennung. Als Phasentrenngefäße sind beispielsweise Standardapparaturen und Standardmethoden geeignet, welche sich zum Beispiel in E. Müller et al., "Liquid-Liquid Extraction", in Ullmann's Encyclopedia of Industrial Chemistry, 2005, Wiley-VCH Verlag GmbH & Co. KGaA, DOI:10.1002/14356007.b03_06, Kapitel 3 "Apparatus" finden lassen. Im Allgemeinen ist die mit den Ameisensäure/Amin-Addukten sowie dem polaren Lösungsmittel angereicherte Flüssigphase schwerer und bildet die Unterphase.

Die Phasentrennung kann beispielsweise nach Entspannung, beispielsweise auf etwa oder nahe Atmosphärendruck, und Abkühlung des flüssigen Reaktionsgemischs, beispielsweise auf etwa oder nahe Umgebungstemperatur,

erfolgen. Allerdings ist dabei zu befürchten, dass zumindest ein Teil des bei dem höherem Reaktionsdruck in den Flüssigphasen gelöste Gas, insbesondere Kohlendioxid, bei der Entspannung entgast und als Gasstrom separat zu verdichten und zum Hydrierreaktor rückzuführen ist. Ebenso ist auch die Unterphase vor der Rückführung zum Hydrierreaktor separat auf Reaktionsdruck zu bringen. Dabei ist für die rückzuführende Gas- und Flüssigphase jeweils ein geeigneter, entsprechend der zu überwindenden Druckdifferenz ausgelegter Verdichter bzw. eine Pumpe vorzusehen, welche im Betrieb zudem zusätzliche Energie verbraucht und auch mit höheren Apparatekosten einhergeht.

Im Rahmen der vorliegenden Erfindung wurde gefunden, dass sich bei dem vorliegenden System, das heißt, einer mit den Ameisensäure/Amin-Addukten sowie dem polaren Lösungsmittel angereicherten Unterphase und einer mit dem tertiären Amin und im Falle des Einsatzes eines homogenen Katalysators auch mit diesem angereicherten Oberphase, beide Flüssigphasen sich auch bei einem deutlich erhöhten Druck sehr gut voneinander trennen können. Daher ist beim erfindungsgemäßen Verfahren das Lösungsmittel bevorzugt so auszuwählen, dass die Trennung der einen, mit den Ameisensäure/Amin-Addukten und dem polaren Lösungsmittel angereicherte Unterphase von der anderen, mit dem tertiären Amin angereicherten Oberphase, sowie die Rückführung der Oberphase zum Hydrierreaktor bei einem Druck von 1 bis 30 MPa abs stattfinden kann. Gemäß dem Gesamtdruck im Hydrierreaktor, liegt der Druck bei bevorzugt höchstens 15 MPa abs. So ist es sogar möglich, ohne vorhergehende Entspannung beide Flüssigphasen voneinander zu trennen und die Oberphase ohne nennenswerte Druckerhöhung zum Hydrierreaktor zurückzuführen. In diesem Falle, sowie auch im Falle einer nur geringfügigen Entspannung ist es möglich, auf eine Rückführung einer etwaigen Gasphase gänzlich zu verzichten. Ob dieser Verzicht für das jeweils konkrete System möglich ist, ist im Zweifel vorab durch einfache experimentelle Beispiele zu ermitteln.

Das erfindungsgemäße Verfahren kann somit in einer Ausführungsform in der Weise durchgeführt werden, dass der Druck und die Temperatur im Hydrierreaktor und im Phasentrenngefäß gleich oder annähernd gleich sind, wobei unter annähernd gleich vorliegend ein Druckunterschied von bis zu +/- 5 bar bzw. ein Temperaturunterschied von bis zu +/- 5 °C verstanden wird.

In einer Ausführungsform erfolgt die Phasentrennung bei einem Druck von mindestens 50%, ganz besonders bevorzugt von mindestens 90% und insbesondere von mindestens 95% des Reaktionsdrucks. Der Druck bei der Phasentrennung liegt besonders bevorzugt bei höchstens 105% und ganz besonders bevorzugt bei höchstens 100% des Reaktionsdrucks.

Überraschenderweise wurde auch gefunden, dass sich bei dem vorliegenden System beide Flüssigphasen auch bei einer erhöhten Temperatur, die der Reaktionstemperatur entspricht, sehr gut voneinander trennen können. Insofern ist zur Phasentrennung auch keine Abkühlung und anschließende Aufheizung der rückzuführenden Oberphase erforderlich, was ebenfalls Energie einspart.

Der Großteil des polaren Lösungsmittels der abgetrennten Unterphase wird in einer Destillationseinheit thermisch von den Ameisensäure/Amin-Addukten abgetrennt, wobei das destillativ entfernte polare Lösungsmittel zum Hydrierreaktor rückgeführt wird. Im Sumpf der Destillationseinheit fallen die reinen Ameisensäure/Amin-Addukte sowie freies Amin an, da sich bei der Entfernung des polaren Lösungsmittels Ameisensäure/Amin-Addukte mit niedrigerem Amingehalt bilden, wodurch sich ein zweiphasiges Sumpfgemisch bestehend aus einer Amin- und einer Ameisensäure/Amin-Addukt-Phase bildet.

Die thermische Trennung des polaren Lösungsmittels oder -gemisches, siehe oben, erfolgt bevorzugtermaßen bei einer Sumpftemperatur, bei welcher bei gegebenem Druck sich keine freie Ameisensäure aus dem Ameisensäure/Amin-Addukt mit dem höheren (x1) oder niedrigerem (x2) Amingehalt bildet. Im Allgemeinen beträgt die Sumpftemperatur der thermischen Abtrenneinheit mindestens 20°C, bevorzugtermaßen mindestens 50°C und besonders bevorzugtermaßen mindestens 70°C sowie im Allgemeinen höchstens 210°C, bevorzugtermaßen höchstens 190°C. Der Druck beträgt dabei im Allgemeinen mindestens 1 hPa abs, bevorzugt mindestens 50 hPa abs und besonders bevorzugt mindestens 100 hPa abs sowie im Allgemeinen höchstens 1 MPa abs und bevorzugtermaßen 0,1 MPa abs.

Die thermische Abtrennung des polaren Lösungsmittels oder -gemisches erfolgt entweder in einem Verdampfer oder in einer Destillationseinheit, bestehend aus Verdampfer und Kolonne, gefüllt mit Packung, Füllkörpern und/oder Böden. Das Lösungsmittel kann nach der thermischen Trennung kondensiert werden, wobei wiederum die dabei freiwerdende Kondensationsenthalpie genutzt werden kann, um beispielsweise das aus der Extraktion kommende Lösungsmittel mit Amin/Ameisensäureaddukt-Gemisch auf Abdampftemperatur vorzuwärmen.

Alternativ können auch nur Teile des Lösungsmittelgemisches abgetrennt werden. Dies gilt insbesondere für Lösungsmittelkomponenten, die in der späteren Ameisensäuredestillation über einen Seitenstrom abgetrennt werden können. (Stichwort wässrige Ameisensäure).

Die Ameisensäure/Amin-Addukte, die nach der thermischen Abtrennung des polaren Lösungsmittels oder -gemisches oder Teilen des Lösungsmittels anfallen, werden dann in einer Destillationseinheit thermisch in freie Ameisensäure und freies tertiäres Amin gespalteten, wobei die gebildete freie Ameisensäure destillativ entfernt und das im Sumpf der Destillationseinheit enthaltene freie tertiäre Amin zum Hydrierreaktor rückführt wird. Dabei kann das bei der thermischen Abtrennung des polaren Lösungsmittels als Zweitphase anfallende freie Amin zuvor in einem Phasentrenngefäß abgetrennt werden, in einem gemeinsamen Phasentrenngefäß zusammen mit dem Sumpfprodukt der thermischen

Spalteinheit zur Ameisensäureabtrennung oder als zweiphasiges Gemisch direkt der Spalteinheit zugeführt werden (siehe allgemeine Ausführungsformen). Die Entnahme der freigesetzten Ameisensäure kann dabei beispielsweise (i) über Kopf, (ii) über Kopf und als Seitenabzug oder (iii) nur als Seitenabzug erfolgen. Wird Ameisensäure über Kopf entnommen, so ist dabei eine Ameisensäure-Reinheit von bis zu 99,99 Gew.-% möglich. Bei einer Entnahme als Seitenabzug wird wässrige Ameisensäure erhalten, wobei hierbei das Gemisch mit etwa 85 Gew.-% Ameisensäure in der Praxis von besonderer Bedeutung ist. Je nach dem Wassergehalt des Zulaufs zur Destillationseinheit wird die Ameisensäure verstärkt als Kopfprodukt oder als Seitenprodukt entnommen. Bei Bedarf ist es sogar möglich, Ameisensäure nur als Seitenprodukt zu entnehmen, wobei dann gegebenenfalls die erforderliche Wassermenge sogar noch explizit zugegeben werden kann. Die thermische Spaltung des Ameisensäure/Amin-Addukts erfolgt im Allgemeinen nach den im Stand der Technik bekannten Verfahrensparametern hinsichtlich Druck, Temperatur sowie apparativer Ausgestaltung. So sei beispielsweise auf die Beschreibungen in EP 0 181 078 A oder WO 2006/021,411 verwiesen. Die einzusetzende Destillationseinheit umfasst in der Regel eine Destillationskolonne, welche im Allgemeinen Füllkörper, Packungen und/oder Böden enthält.

Im Allgemeinen beträgt die Sumpftemperatur in der Destillationskolonne mindestens 130°C, bevorzugt mindestens 140°C und besonders bevorzugt mindestens 150°C, sowie im Allgemeinen höchstens 210°C, bevorzugt höchstens 190°C und besonders bevorzugt höchstens 185°C. Der Druck beträgt im Allgemeinen mindestens 1 hPa abs, bevorzugt mindestens 50 hPa abs und besonders bevorzugt mindestens 100 hPa abs, sowie im Allgemeinen höchstens 500 hPa abs, bevorzugt höchstens 300 hPa abs und besonders bevorzugt höchstens 250 hPa abs.

Gegebenenfalls wird als Seitenprodukt noch ein wasserhaltiger Strom an Ameisensäure entnommen. Bei einem Zusatz von Wasser, beispielsweise zur Förderung der Hydrierung oder der Katalysatorextraktion, ist dies sogar besonders vorteilhaft.

Die Lösung des Addukts aus tertiärem Amin und Ameisensäure wird mit aus den entsprechenden Phasentrenngefäßen stammendem Strömen von freiem tertiärem Amin extrahiert und in den Hydrierreaktor rückgeführt. Dies geschieht, um Restmengen an Hydrierkatalysator aus dem Produktstrom abzutrennen. Ohne diese Extraktion könnte Hydrierkatalysator in die Vorrichtung zur thermischen Spaltung des Addukts aus tertiärem Amin und Ameisensäure gelangen, die Zersetzung von Ameisensäure katalysieren und so die Ameisensäureausbeute mindern. Restmengen aus Wasserstoff und Kohlendioxid werden als Abgas entsorgt.

Die Extraktion erfolgt bei Temperaturen von 30 bis 100 °C und Drücken von 1 bis 80 bar. Die Extraktion kann auch unter Wasserstoffdruck durchgeführt werden.

Die Extraktion des Hydrierkatalysators kann in jeder geeigneten, dem Fachmann bekannten Vorrichtung durchgeführt werden, bevorzugt in Gegenstrom-Extraktionskolonnen, Mixer-Settler-Kaskaden oder Kombinationen von Mixer-Settlern mit Kolonnen.

Der Produktstrom aus der Extraktionsvorrichtung, der aus einer Lösung des Ameisensäure/Amin-Addukts in dem jeweiligen Lösungsmittel oder Lösungsmittelgemisch besteht, wird in die thermische Spalteinheit geleitet, um das polare Lösungsmittel oder - gemisch vom Ameisensäure/Amin-Addukt zu trennen. Die aus der Extraktionsvorrichtung tertiäres Amin und Hydrierkatalysator enthaltende Phase wird in die Hydrierstufe zurückgeführt.

Unter Umständen werden neben dem Katalysator Anteile einzelner Komponenten des polaren Lösungsmittels aus der zu extrahierenden Flüssigphase im Extraktionsmittel, dem Aminstrom, gelöst. Dies ist für das Verfahren kein Nachteil, da die bereits extrahierte Menge an Lösungsmittel nicht der Lösungsmittelabtrennung zugeführt werden muss und somit unter Umständen Verdampfungsenergie und Apparatekosten einspart.

Es kann vorteilhaft sein, zwischen der Extraktionsapparatur und der thermischen Trennvorrichtung eine Vorrichtung zur Adsorption von Spuren von Hydrierkatalysator zu integrieren. Zahlreiche Adsorptionsmittel sind für die Adsorption geeignet. Beispiele hierfür sind Polyacrylsäure und Salze dieser, sulfonierte Polystyrole und Salze dieser, Aktivkohlen, Montmorillonite, Bentonite, Kieselgele sowie Zeolithe.

Falls die Menge an Hydrierkatalysator im Produktstrom des Phasentrenngefäßes B kleiner als 1 ppm, insbesondere kleiner als 0.1 ppm ist, reicht die Adsorptionsvorrichtung für die Abtrennung des Hydrierkatalysators und seine Rückgewinnung aus. Dann kann die Extraktionsstufe entfallen und das tertiäre Amin zusammen mit dem organischen Lösungsmittel in die Hydrierstufe zurückgeführt werden.

Das erfindungsgemäße Verfahren besitzt gegenüber den in EP 181.078 B1 und EP 357.243 B1 vorbeschriebenen integrierten Verfahren eine Reihe von Vorteilen: Für das Abfangen der Ameisensäure in der Hydrierung und die thermische Spaltung der Ameisensäure/Amin-Addukte wird das gleiche tertiäre Amin verwendet. Dieses Amin, das bei der thermischen Spaltung frei anfällt, wird dann zur Extraktion von Katalysatorresten in der Produktphase eingesetzt werden, um Spuren des Katalysators mit dem Amin in das Reaktionsgefäß zurückzuführen. Es besitzt eine höhere Stabilität als die vorbeschriebenen N-Alkylimidazole. Verluste an Edelmetallen treten praktisch nicht auf. Es wird verhindert, dass der Katalysator in die thermische Spalteinheit gelangt und darin die Zersetzung von Ameisensäure katalysiert. Von großem Vorteil ist, dass er in seiner aktiven Form abgetrennt und zurückgeführt werden kann. Es werden hohe Ameisensäure-Ausbeuten und eine hohe Produktreinheit erzielt. An die Stelle von zwei Destillationen treten Extraktionen und Phasentrennungen. Hierdurch werden Energie- und Investitionskosten gesenkt.

Das erfindungsgemäße Verfahren ermöglicht die Gewinnung von konzentrierter Ameisensäure in hoher Ausbeute und hoher Reinheit durch Hydrierung von Kohlendioxid. Es zeichnet sich insbesondere durch eine besonders einfache Verfahrensführung aus, welche gegenüber dem Stand der Technik ein einfacheres Verfahrenskonzept, einfachere Verfahrensstufen, eine geringere Anzahl an Verfahrensstufen sowie einfachere Apparate aufweist. So wird beispielsweise durch geeignete Wahl des tertiären Amins und des polaren Lösungsmittels im Falle des Einsatzes eines homogenen Katalysators dieser durch Phasentrennung von Ameisensäure/Amin-Addukten getrennt und ohne weitere Aufarbeitungsschritte zum Hydrierreaktor rückgeführt. Die Phasentrennung kann dabei auch unter Druck erfolgen. Aufgrund der zügigen Abtrennung des Katalysators von den gebildeten Ameisensäure/Amin-Addukten wird eine Rückreaktion unter Zersetzung in Kohlendioxid und Wasserstoff unterdrückt. Zudem werden durch die Rückhaltung beziehungsweise Abtrennung des Katalysators aufgrund der Ausbildung zweier Flüssigphasen Verluste an Katalysator und damit Verluste an Edelmetall minimiert. Darüber hinaus kann im Produktstrom verbliebener Katalysator durch die Extraktion mit dem freien Amin aus den thermischen Spalteinheiten nahezu vollständig in den Hydrierreaktor rückgeführt werden, was die Verluste an Edelmetall weiter minimiert und für einen wirtschaftlichen Prozess von sehr großem Vorteil ist. Zudem wird die Zersetzung der Ameisensäure in den thermischen Spalteinheiten weitgehend unterdrückt wird, ohne dass der Katalysator desaktiviert werden musste. Ferner ist beim erfindungsgemäßen Verfahren kein aufwändiger, separater Basenaustausch erforderlich, so dass die im Hydrierreaktor gebildeten Ameisensäure/Amin-Addukte direkt zur thermischen Spaltung eingesetzt werden können. Der Einsatz eines niedrigsiedenden polaren Lösungsmittels ermöglicht dessen thermische Abtrennung in einer der thermischen Spaltung der Ameisensäure vorgelagerten Stufe unter milden Bedingungen, wodurch die Veresterung eingesetzter Alkohole und die Zersetzung der Ameisensäure minimiert wird, ein geringerer Energiebedarf nötig ist und eine höhere Reinheit der Ameisensäure erzielt werden kann. Als Folge des einfacheren Verfahrenskonzepts ist die zur Durchführung des erfindungsgemäßen Verfahrens erforderliche Produktionsanlage gegenüber dem Stand der Technik kompakter im Sinne eines geringeren Platzbedarfs und des Einsatz von wenigeren Apparaten. Sie weist einen geringeren Investitionsaufwand und einen geringeren Energiebedarf auf.

Die Erfindung wird im Folgenden anhand von Ausführungsbeispielen sowie einer Zeichnung näher erläutert.

Beispiele A-1 bis A-16 (erfindungsgemäß) (Hydrierung und Phasentrennung, Zugabe von Wasser nach der Reaktion)

Ein 250 mL Autoklav aus Hastelloy C, ausgestattet mit einem Magnetrührstab, wurde unter Inertbedingungen mit tertiärem Amin, polarem Lösungsmittel und homogenem Katalysator befüllt. Anschließend wurde der Autoklav verschlossen und bei Raumtemperatur $CO_2$ aufgepresst. Nachfolgend wurde $H_2$ aufgepresst und der Reaktor unter Rühren (700 U/min) erwärmt. Nach der gewünschten Reaktionszeit wurde der Autoklav abgekühlt und das Reaktionsgemisch entspannt. Es wurde - sofern nicht anders vermerkt - nach der Wasserzugabe ein zweiphasiges Produktgemisch erhalten, wobei die Oberphase mit dem noch freien tertiären Amin und dem homogenen Katalysator, und die Unterphase mit dem polaren Lösungsmittel, Wasser und den gebildeten Ameisensäure/Amin-Addukten angereichert war. Der Gesamtgehalt an Ameisensäure im Ameisensäure/Amin-Addukt wurde durch Titration mit 0,1 N KOH in MeOH potentiometrisch mit einem "Mettler Toledo DL50"-Titrator bestimmt. Daraus wurden die Turnover. Frequency (=TOF; zur Definition der TOF siehe: J.F. Hartwig, Organotransition Metal Chemistry, 1. Auflage, 2010, Universtiy Science Books, Sausalito/California S.545) sowie die Reaktionsrate berechnet. Der Rutheniumgehalt wurde per Atom-AbsorptionsSpektroskopie bestimmt. Die Zusammensetzung der beiden Phasen wurde durch Gaschromatographie sowie Protonen-NMR-Spektroskopie bestimmt. Die Parameter und Ergebnisse der einzelnen Versuche sind in Tabelle 1.1 bis 1.5 wiedergegeben.

Bei den Ausführungsformen in den Versuchen A-1 - bis A-16 liegen nach der Reaktion ungünstige Ru-Verteilungskoeffizienten $k_{Ru}$ vor. Die Produktphase, Strom 3, wurde deshalb anschließend mit Wasser versetzt, wobei sich ein zweiphasiges Gemisch bildete, wobei die Oberphase hauptsächlich aus Amin und dem Alkohol und die Unterphase aus den Ameisensäure-Amin-Addukten, dem Alkohol und Wasser bestand, wobei sich durch die Wasserzugabe verbesserte Ru-Verteilungskoeffizienten zwischen diesen beiden Phasen einstellten. Bei den Ausführungsformen in den Vergleichsversuchen zu A3, A7 und A15 (Versuchen A-17 bis A-19 in Tabelle 1.6) wurde die gesamte Menge an Wasser schon bei der Reaktion zugegeben. Hier lässt sich klar erkennen, das bei hier eingesetzten Lösungsmitteln und Katalysatoren die Zugabe dieser Wassermenge bei der Hydrierung zu schlechteren Rutheniumverteilungskoeffizienten nach der Reaktion und/oder niedrigeren Reaktionsraten führt.

**Tabelle 1.1**

| | Beispiel A-1 | 50 g der Unterphase aus Beispiel A-2 werden mit 6.1 g Wasser versetzt. Dabei bilden sich zwei Phasen aus. | Beispiel A-2 | 50 g der Unterphase aus Beispiel A-2 werden mit 8.3 g Wasser versetzt. Dabei bilden sich zwei Phasen aus. |
|---|---|---|---|---|
| Tertiäres Amin | 75 g Trihexylamin | | 75 g Tripentylamin | |
| Polares Lösungsmittel (eingesetzt) | 25 g Methanol | | 25 g Methanol | |
| Katalysator | 0.18 g $[Ru(P^nBu_3)_4(H)_2]$ | | 0.18 g $[Ru(P^nBu_3)_4(H)_2]$ | |
| Aufpressen von $CO_2$ | Mit 19.9 g auf 1.8 MPa abs | | Mit 19.7 g auf 1.9 MPa abs | |
| Aufpressen von $H_2$ | Auf 9.8 MPa abs | | Auf 9.9 MPa abs | |
| Erwärmen | Auf 50°C | | Auf 50°C | |
| Druckänderung | Auf 9.4 MPa abs | | Auf 9.4 MPa abs | |
| Reaktionszeit | 1 Stunde | | 1 Stunde | |
| Besonderheit | - | | - | |
| Oberphase | 15.9 g<br>12.4 % Methanol<br>87.6 % Trihexylamin | 18.8 g<br>2.8 % Methanol<br>97.2 % Trihexylamin | 38.5 g<br>4.9 % Methanol<br>95.1 % Tripentylamin | 10.4 g<br>1.1 % Methanol<br>98.9 % Tripentylamin |
| Unterphase | 87.3 g<br>5.9 % Ameisensäure<br>26.4 % Methanol<br>67.7 % Trihexylamin | 36.2 g<br>7.3 % Ameisensäure<br>16.9 % Wasser<br>35.0 % Methanol<br>40.8 % Trihexylamin | 64.1 g<br>8.1 % Ameisensäure<br>36.1 % Methanol<br>55.8 % Tripentylamin | 46.7 g<br>8.0 % Ameisensäure<br>17.8 % Wasser<br>38.5 % Methanol<br>35.7 % Tripentylamin |
| $K_{Ru}$ ($c_{Ru}$ Oberphase / $C_{Ru}$ Unterphase) | 0.3 | 4.0 | 0.7 | 2.7 |
| TOF | 560 $h^{-1}$ | - | 562 $h^{-1}$ | - |
| Reaktionsrate | 1.09 mol $kg^{-1}$ $h^{-1}$ | - | 1.09 mol $kg^{-1}$ $h^{-1}$ | - |

**Tabelle 1.2**

| | Beispiel A-3 | 50 g der Unterphase aus Beispiel A-3 werden mit 5.7 g Wasser versetzt. Dabei bilden sich zwei Phasen aus. | Beispiel A-4 | 50 g der Unterphase aus Beispiel A-4 werden mit 7.8 g Wasser versetzt. Dabei bilden sich zwei Phasen aus. |
|---|---|---|---|---|
| Tertiäres Amin | 75 g Trihexylamin | | 75 g Tripentylamin | |
| Polares Lösungsmittel (eingesetzt) | 24 g Methanol<br>1 g Wasser | | 24 g Methanol<br>1 g Methanol | |
| Katalysator | 0.18 q [$Ru(P^nBu_3)_4(H)_2$] | | 0.18 g [$Ru(P^nBu_3)_4(H)_2$] | |
| Aufpressen von $CO_2$ | Mit 20.1 g auf 2.1 MPa abs | | Mit 20.1 g auf 2.2 MPa abs | |
| Aufpressen von $H_2$ | Auf 10.1 MPa abs | | Auf 10.2 MPa abs | |
| Erwärmen | Auf 50°C | | Auf 50°C | |
| Druckänderung | Auf 9.6 MPa abs | | Auf 10.3 MPa abs | |
| Reaktionszeit | 1 Stunde | | 1 Stunde | |
| Besonderheit | - | | - | |
| Oberphase | 27.4 g<br>9.0 % Methanol<br>91.0 % Trihexylamin | 12.3 g<br>2.8 % Methanol<br>97.2 % Trihexylamin | 43.9 g<br>3.4 % Methanol<br>96.6 % Tripentylamin | 7.3 g<br>1.3 % Methanol<br>98.7 % Tripentylamin |
| Unterphase | 76.3 g<br>7.1 % Ameisensäure<br>1.3 % Wasser<br>28.2 % Methanol<br>63.4 % Trihexylamin | 41.7 g<br>7.8 % Ameisensäure<br>15.2 % Wasser<br>33.0 % Methanol<br>44.0 % Trihexylamin | 59.2 g<br>8.7 % Ameisensäure<br>38.0 % Methanol<br>1.7 % Wasser<br>51.6 % Tripentylamin | 49.1 g<br>8.3 % Ameisensäure<br>17.6 % Wasser<br>38.5 % Methanol<br>35.6 % Tripentylamin |
| $K_{Ru}$ ($c_{Ru}$ Oberphase / $C_{Ru}$ Unterphase) | 0.6 | 3.3 | 1.1 | 1.7 |
| TOF | 591 $h^{-1}$ | - | 551 $h^{-1}$ | - |
| Reaktionsrate | 1.09 mol $kg^{-1}$ $h^{-1}$ | - | 1.08 mol $kg^{-1}$ $h^{-1}$ | - |

**Tabelle 1.3**

| | Beispiel A-5 | Beispiel A-6 | Beispiel A-7 | Beispiel A-8 |
|---|---|---|---|---|
| Tertiäres Amin | 75 g Tripentylamin | 75 g Trihexylamin | 75 g Tripentylamin | 75 g Tripentylamin |
| Polares Lösungsmittel (eingesetzt) | 25 g Methanol | 25 g Methanol | 25 g Methanol | 25 g Methanol |
| Katalysator | 0.16 g [Ru(PnOct3)4(H)2] | 0.16 g [Ru(PnOct3)4(H)2] | 0.16 g [Ru(PnOct3)4(H)2] | 0.33 g [Ru(PnOct3)4(H)2] |
| Aufpressen von CO2 | Mit 30.0 g auf 2.5 MPa abs | Mit 20.0 g auf 1.7 MPa abs | Mit 19.9 g auf 2.1 MPa abs | Mit 20.0 g auf 2.0 MPa abs |

(fortgesetzt)

| | Beispiel A-5 | Beispiel A-6 | Beispiel A-7 | Beispiel A-8 |
|---|---|---|---|---|
| Aufpressen von H2 | Auf 8.0 MPa abs | Auf 9.7 MPa abs | Auf 10.0 MPa abs | Auf 10.0 MPa abs |
| Erwärmen | Auf 50°C | Auf 50°C | Auf 50°C | Auf 50°C |
| Druckänderung | Auf 10.5 MPa abs | Auf 10.4 MPa abs | Auf 10.6 MPa abs | Auf 10.8 MPa abs |
| Reaktionszeit | 1 Stunde | 1 Stunde | 1 Stunde | 1 Stunde |
| Besonderheit | Zugabe von 2 g Wasser nach der Reaktion | Zugabe von 2 g Wasser nach der Reaktion | Zugabe von 5 g Wasser nach der Reaktion | Zugabe von 3 g Wasser nach der Reaktion |
| Oberphase | 55.9 g<br>4.9 % Methanol<br>95.1 % Tripentylamin | 43.9 g<br>9.7 % Methanol<br>92.3 % Trihexylamin | 55.5 g<br>3.1 % Methanol<br>96.9 % Tripentylamin | 40.5 g<br>VH10-44 |
| Unterphase | 44.6 g<br>6.4 % Ameisensäure<br>4.5 % Wasser<br>50.0 % Methanol<br>39.1 % Tripentylamin | 49.9 g<br>5.7 % Ameisensäure<br>41.5 % Methanol<br>4.0 % Wasser<br>48.8 % Trihexylamin | 45.5 g<br>6.1 % Ameisensäure<br>51.2 % Methanol<br>11.0 % Wasser<br>31.7 % Tripentylamin | 61.5 g<br>7.0 % Ameisensäure<br>35.9 % Methnaol<br>4.9 % Wasser<br>52.2 % Trihexylamin |
| KRu (cRu Oberphase / cRu Unterphase) | 4.2 | 6.8 | 10.9 | 41.0 |
| TOF | 602 h-1 | 602 h-1 | 586 h-1 | 447 h-1 |
| Reaktionsrate | 0.62 mol kg-1 h-1 | 0.62 mol kg-1 h--1 | 0.60 mol kg-1 h-1 | 0.91 mol kg-1 h-1 |

**Tabelle 1.4**

| | Beispiel A-9 | Beispiel A-10 | Beispiel A-11 | Beispiel A-12 |
|---|---|---|---|---|
| Tertiäres Amin | 75 g Trihexylamin | 75 g Trihexylamin | 75 g Trihexylamin | 75 g Trihexylamin |
| Polares Lösungsmittel (eingesetzt) | 25 g Methanol | 25 g Methanol | 25 g Methanol | 25 g Methanol |
| Katalysator | 0.16 g [Ru(P*n*Octyl$_3$)$_4$(H)$_2$], 0.08 g 1,2-Bis(dicyclohexylphosphino) ethan | 0.16 g [Ru(P*n*Octyl$_3$)$_4$(H)$_2$) | 0.16 g [Ru(P*n*Octyl$_3$)$_4$(H)$_2$]-0.08 g 1,2-Bis(dicyclohexylphosphino )ethan | 0.32 g [Ru(P*n*Octyl$_3$)$_4$(H)$_2$], 0.17 g 1,2-Bis(dicyclohexylphosphin o)ethan, 0.15 g P*n*Octyl$_3$ |
| Aufpressen von $CO_2$ | Auf 2.0 MPa abs | Auf 1.9 MPa abs | Auf 1.8 MPa abs | Auf 2.0 MPa abs |
| Aufpressen von $H_2$ | Auf 10.0 MPa abs | Auf 9.9 MPa abs | Auf 9.8 MPa abs | Auf 12.0 MPa abs |
| Erwärmen | 70°C | 70°C | 40°C | 50°C |
| Druckänderung | Auf 10.4 MPa abs | Auf 11.1 MPa abs | Auf 10.0 MPa abs | Auf 12.4 MPa abs |
| Reaktionszeit | 1 Stunde | 1 Stunde | 1 Stunde | 1 Stunde |
| Besonderheit | Zugabe von 5 g Wasser nach der Reaktion | Zugabe von 5 g Wasser nach der Reaktion | Zugabe von 5 g Wasser nach der Reaktion | Zugabe von 5 g Wasser nach der Reaktion |
| Oberphase | 42.5 g | 55.2 g | 50.5 g | 25.9 g |
| Unterphase | 58.4 g<br>7.3 % Ameisensäure | 43.4 g<br>5.4 % Ameisensäure | 49.0 g<br>6.2 % Ameisensäure | 78.0 g<br>8.5 % Ameisensäure |
| $K_{Ru}$ ($C_{Ru}$ Oberphase / $C_{Ru}$ Unterphase) | 16.3 | 100 | 2.2 | 19.4 |
| TOF | 904 $h^{-1}$ | 492 $h^{-1}$ | 650 $h^{-1}$ | 696 |
| Reaktionsrate | 0.92 mol $kg^{-1}$ $h^{-1}$ | 0.52 mol $kg^{-1}$ $h^{-1}$ | 0.62 mol $kg^{-1}$ $h^{-1}$ | 1.38 mol $kg^{-1}$ $h^{-1}$ |

**Tabelle 1.5**

| | Beispiel A-13 | Beispiel A-14 | Beispiel A-15 | Beispiel A-16 |
|---|---|---|---|---|
| Tertiäres Amin | 75 g Trihexylamin | 75 g Trihexylamin | 75 g Trihexylamin | 75 g Trihexylamin |
| Polares Lösungsmittel (eingesetzt) | 25 g Methanol | 25 g Methanol | 25 g Methanol | 25 g Ethanol |
| Katalysator | 0.11 g $[Ru(COD)Cl_2]_2$, 0.17 g 1,2-Bis(dicyclohexylphosphino) ethan, 0.15 g P*n*$Oct_3$ | 0.32g [Ru(P*n*$Octyl_3)_4(H)_2$], 0.08 g 1,2-Bis(diphenylphosphino) ethan | 0.32 g [Ru(P*n*$Octyl_3)_4(H)_2$], 0.08 g 1,2-Bis(dicyclohexylphosphino) ethan | 0.18 g $[Ru(P^nOCt_3)_4(H)_2]$ |
| Aufpressen von $CO_2$ | Auf 1.6 MPa abs | Auf 1.6 MPa abs | Auf 1.7 MPa abs | Mit 20.0 g auf 2.2 MPa abs |
| Aufpressen von $H_2$ | Auf 12.0 MPa abs | Auf 9.6 MPa abs | Auf 9.7 MPa abs | Auf 10.2 MPa abs |
| Erwärmen | 50°C | 50°C | 50°C | Auf 50°C |
| Druckänderung | Auf 12.1 MPa abs | Auf 8.6 MPa abs | Auf 9.5 MPa abs | Auf 11.1 MPa abs |
| Reaktionszeit | 1 Stunde | 2 Stunden | 2 Stunden | 1 Stunde |
| Besonderheit | Zugabe von 5 g Wasser nach der Reaktion | Zugabe von 5 g Wasser nach der Reaktion | Zugabe von 5 g Wasser nach der Reaktion | Einphasiger Reaktionsaustrag; Zugabe von 5 g Wasser nach der Reaktion, wobei sich zwei Phasen ausbilden |
| Oberphase | 16.6 g | 36.2 g | 26.7 g | 66.3 g<br>8.8 % Ethanol<br>0.8 % Wasser<br>90.4 % Trihexylamin |
| Unterphase | 88.1 g<br>9.0 % Ameisensäure | 66.1 g<br>7.3 % Ameisensäure | 74.0 g<br>8.3 % Ameisensäure | 29.6 g<br>2.7 % Ameisensäure<br>20.9 % Wasser<br>64.4 % Ethanol<br>12 % Trihexylamin |
| $K_{Ru}$ ($c_{Ru}$ Oberphase / $C_{Ru}$ Unterphase) | 12.0 | 1.4 | 14 | 22.5 |
| TOF | 435 $h^{-1}$ | 258 $h^{-1}$ | 335 $h^{-1}$ | 152 $h^{-1}$ |
| Reaktionsrate | 1.64 mol $kg^{-1}$ $h^{-1}$ | 1.02 mol $kg^{-1}$ $h^{-1}$ | 1.33 mol $kg^{-1}$ $h^{-1}$ | 0.18 mol $kg^{-1}$ $h^{-1}$ |

**Tabelle 1.6: Vergleichsversuche - Wasserzugabe bei der Reaktion**

| | Beispiel A-17 (Vergleichsversuch zu A3) | Beispiel A-18 (Vergleichsversuch zu A15) | Beispiel A-19 (Vergleichsversuch zu A7) |
|---|---|---|---|
| Tertiäres Amin | 75 g Trihexylamin | 75 g Trihexylamin | 75 g Tripentylamin |
| Polares Lösungsmittel (eingesetzt) | 24 g Methanol<br>6.7 g Wasser | 25 g Methanol<br>5.0 g Wasser | 25 g Methanol<br>5.0 g Wasser |
| Katalysator | 0.18 g $[Ru(P^nBu_3)_4(H)_2]$ | 0.32 g $[Ru(P\textit{n}Octyl_3)_4(H)_2]$, 0.08 g 1,2-Bis(dicyclohexylphosphino)e than | 0.16 g $[Ru(P\textit{n}Octyl_3)_4(H)_2$ |
| Aufpressen von $CO_2$ | Mit 20.0 g auf 3.5 MPa abs | Mit 20.0 g auf 2.5 MPa abs | Mit 20.0 g auf 2.1 MPa abs |
| Aufpressen von $H_2$ | Auf 11.5 MPa abs | Auf 10.6 MPa abs | Auf 10.1 MPa abs |
| Erwärmen | Auf 50°C | Auf 50°C | Auf 50°C |
| Druckänderung | Auf 11.0 MPa abs | Auf 11.0 MPa abs | Auf 11.3 MPa abs |
| Reaktionszeit | 1 Stunde | 1 Stunde | 1 Stunde |
| Besonderheit | Wasser wird vor der Reaktion zugesetzt | Wasser wird vor der Reaktion zugesetzt | Wasser wird vor der Reaktion zugesetzt |
| Oberphase | 44.1 g | 64.3 g | 66.6 g |
| Unterphase | 65.9 g<br>7.5 % Ameisensäure | 41.7 g<br>4.7 % Ameisensäure | 37.6 g<br>2.4 % Ameisensäure |
| $K_{Ru}(c_{Ru}$ Oberphase / $c_{Ru}$ Unterphase) | 1.8 | 1.3 | 32.5 |
| TOF | 551 $h^{-1}$ | 394 $h^{-1}$ | 187 $h^{-1}$ |
| Reaktionsrate | 0.98 mol $kg^{-1}$ $h^{-1}$ | 0.4 mol $kg^{-1}$ $h^{-1}$ | 0.19 mol $kg^{-1}$ $h^{-1}$ |

Beispiel B1-B4 (Extraktion des Katalysators);

Ein 100 mL Autoklav aus Hastelloy C, ausgestattet mit einem Blattrührer, wurde unter Inertbedingungen mit dem Trialkylamin, polaren Lösungsmittel und dem Katalysator befüllt. Anschließend wurde der Autoklav verschlossen und bei Raumtemperatur wurden $CO_2$ aufgepresst. Nachfolgend wurde $H_2$ aufgepresst und der Reaktor unter Rühren (1000 U/min) erwärmt. Nach der Reaktionszeit wurde der Autoklav abgekühlt und das Reaktionsgemisch entspannt. Nach der Reaktion wurde zum Reaktionsaustrag Wasser gegeben und 10 Minuten bei Raumtemperatur gerührt. Es wurde ein zweiphasiges Produktgemisch erhalten, wobei die obere Phase mit dem noch freien tertiären Amin und dem homogenen Katalysator, und die untere Phase mit dem polaren Lösungsmittel und dem gebildeten Ameisensäure/Amin-Addukt angereichert war. Die Unterphase wurde abgetrennt und unter Inertbedingungen dreimal mit der gleichen Menge (Masse an Amin entspricht der Masse der Unterphase) frischem Trialkylamin (10 Minuten bei Raumtemperatur rühren und anschließend Phasen trennen). Der Gesamtgehalt an Ameisensäure im Ameisensäure/Amin-Addukt wurde durch Titration mit 0,1 N KOH in MeOH potentiometrisch mit einem "Mettler Toledo DL50"-Titrator bestimmt. Der Rutheniumgehalt wurde per AAS-bestimmt. Die Parameter und Ergebnisse der einzelnen Versuche sind in Tabelle 1.7 wiedergegeben.

Die Beispiele B-1 bis B-4 zeigen, dass sich durch Variation des Katalysators und der zugegebenen Wassermenge bei der Bildung von Ameisensäure das Ruthenium in der Produktphase auf Werte von bis zu unter einem ppm Ruthenium abreichern lässt.

**Tabelle 1.7**

| | Beispiel B-1 | Beispiel B-2 | Beispiel B-3 | Beispiel B-4 |
|---|---|---|---|---|
| Tertiäres Amin | 37.5 g Trihexylamin | 37.5 g Trihexylamin | 37.5 g Trihexylamin | 37.5 g Trihexylamin |

(fortgesetzt)

| | Beispiel B-1 | Beispiel B-2 | Beispiel B-3 | Beispiel B-4 |
|---|---|---|---|---|
| Polares Lösungsmittel (eingesetzt) | 12.0 g Methanol | 12.0 g Methanol | 12.0 g Methanol | 12.0 g Methanol<br>0.5 g Wasser |
| Katalysator | 0.16 g [Ru(P*n*Octyl$_3$)$_4$(H)$_2$] | 0.16 g [Ru(P*n*Octyl$_3$)$_4$(H)$_2$] | 0.16 g [Ru(P*n*Octyl$_3$)$_4$(H)$_2$] | 0.1 g [Ru(P*n*Butyl$_3$)$_4$(H)$_2$] |
| Aufpressen von $CO_2$ | Auf 1.7 MPa abs | Auf 1.6 MPa abs | Auf 1.8 MPa abs | Auf 1.7 MPa abs |
| Aufpressen von $H_2$ | Auf 8.0 MPa abs | Auf 8.0 MPa | Auf 8.0 MPa | Auf 8.0 MPa |
| Erwärmen | 50°C | 50°C | 50°C | 50°C |
| Reaktionszeit | 1.5 Stunden | 1.5 Stunden | 16 Stunden | 1.5 Stunden |
| Wasserzugabe nach der Reaktion | 2.5 g | 4.7 g | 2.5 g | 0.8 g |
| Oberphase | 26.3 g | 27.4 g | 23.2 g | 17.5 g |
| Unterphase | 24.7 g<br>6.6 % Ameisensäure | 25.5 g<br>5.9 % Ameisensäure | 28.1 g<br>6.8 % Ameisensäure | 28.9 g<br>7.4 % Ameisensäure |
| $c_{Ru}$ Oberphase nach Reaktion und Wasserzugabe | 350 ppm | 280 ppm | 370 ppm | 200 ppm |
| $c_{Ru}$ Unterphase nach Ex-traktion | 4 ppm | 2 ppm | < 1 ppm | 43 ppm |

Beispiel C1-9 (Wiederverwendung des Katalysators und Katalysatorextraktion);

Ein 100 mL Autoklav aus Hastelloy C, ausgestattet mit einem Blattrührer, wurde unter Inertbedingungen mit dem Trialkylamin, polaren Lösungsmittel und dem Katalysator befüllt. Anschließend wurde der Autoklav verschlossen und bei Raumtemperatur wurden $CO_2$ aufgepresst. Nachfolgend wurde $H_2$ aufgepresst und der Reaktor unter Rühren (1000 U/min) erwärmt. Nach der Reaktionszeit wurde der Autoklav abgekühlt und das Reaktionsgemisch entspannt. Nach der Reaktion wurde zum Reaktionsaustrag Wasser gegeben und 10 Minuten bei Raumtemperatur gerührt. Es wurde ein zweiphasiges Produktgemisch erhalten, wobei die obere Phase mit dem noch freien tertiären Amin und dem homogenen Katalysator, und die untere Phase mit dem polaren Lösungsmittel und dem gebildeten Ameisensäure/Amin-Addukt angereichert war. Die Phasen wurden anschließend getrennt der Ameisensäuregehalt der Unterphase sowie der Rutheniumgehalt beider Phasen nach den unten beschriebenen Methoden bestimmt. Die Oberphase, enthaltend Rutheniumkatalysator, wurde dann mit frischem Amin auf 37.5 g ergänzt und mit dem gleichen Lösungsmittel unter den gleichen Reaktionsbedingungen wie zuvor erneut zur $CO_2$-Hydrierung eingesetzt. Nach vollendeter Reaktion und Wasserzugabe wurde die Unterphase abgetrennt und unter Inertbedingungen dreimal mit der gleichen Menge (Masse an Amin entspricht der Masse der Unterphase) frischem Trialkylamin (10 Minuten bei Raumtemperatur rühren und anschließend Phasen trennen) zur Katalysatorextraktion versetzt. Der Gesamtgehalt an Ameisensäure im Ameisensäure/Amin-Addukt wurde durch Titration mit 0,1 N KOH in MeOH potentiometrisch mit einem "Mettler Toledo DL50"-Titrator bestimmt. Der Rutheniumgehalt wurde per AAS-bestimmt. Die Parameter und Ergebnisse der einzelnen Versuche sind in den Tabellen 1.8 bis 1.13 wiedergegeben.

Die Beispiele C-1 bis C-9 zeigen, dass sich durch Variation des Katalysators, der zugegebenen Wassermenge (sowohl vor als auch nach der Reaktion) und der Reaktionsbedingungen der aktive Katalysator wieder für die $CO_2$-Hydrierung einsetzten läßt und sich das Ruthenium in der Produktphase mit nur einmaliger Extraktion bis auf 2 ppm abreichern lässt.

**Tabelle 1.8**

| | Beispiel C-1a (erste Hydrierung) | Beispiel C-1b (Wiederverwendung des Katalysators und Extraktion) | Beispiel C-2a (erste Hydrierung) | Beispiel C-2b (Wiederverwendung des Katalysators und Extraktion) |
|---|---|---|---|---|
| Tertiäres Amin | 37.5 g Trihexylamin | Oberphase aus C-1a auf 37.5 g mit frischem Trihexylamin ergänzt | 37.5 g Trihexylamin | Oberphase aus C-2a auf 37.5 g mit frischem Trihexylamin ergänzt |
| Polares Lösungsmittel (einqesetzt) | 12.0 g Methanol<br>0.5 g Wasser | 12.0 g Methanol | 12.0 g Methanol<br>0.3 g Wasser | 12.0 g Methanol<br>0.25 g Wasser |
| Katalysator | 0.16 g [$Ru(PnOctyl_3)_4(H)_2$], 0.08 g 1,2-Bis(dicyclohexylphosphino)ethan | Oberphase aus C-1a | 0.16 g [$Ru(PnOctyl_3)_4(H)_2$], 0.08 g 1,2-Bis(dicyclohexylphosphino)ethan | Oberphase aus C-2a |
| Aufpressen von $CO_2$ | Auf 1.6 MPa abs | Auf 1.5 MPa abs | Auf 1.6 MPa abs | Auf 1.7 MPa abs |
| Aufpressen von $H_2$ | Auf 8.0 MPa abs | Auf 8.0 MPa | Auf 8.0 MPa abs | Auf 8.0 MPa abs |
| Erwärmen | 70°C | 70°C | 70°C | 70°C |
| Reaktionszeit | 1.5 Stunden | 1.5 Stunden | 1.5 Stunden | 1.5 Stunden |
| Wasserzugabe nach der Reaktion | 0.5 g | 1.0 g | 1.0 g | 1.0 g |
| Oberphase | 19.3 g | 24.7 g | 20.9 g | 25.9 9. |
| Unterphase | 30.8 g<br>6.0 % Ameisensäure | 25.4 g<br>5.9 % Ameisensäure | 29.9 9<br>6.6 % Ameisensäure | 24.8 g<br>6.4 % Ameisensäure |
| $c_{Ru}$ Oberphase nach Reaktion und Wasserzugabe | 250 ppm | 170 ppm | 200 ppm | 140 ppm |
| $c_{Ru}$ Unterphase nach Reaktion und Wasserzugabe | 120 ppm | - | 130 ppm | - |
| $c_{Ru}$ Unterphase nach Extraktion | - | 9 ppm | - | 10 ppm |

**Tabelle 1.9**

| | Beispiel C-3a (erste Hydrierung) | Beispiel C-3b (Wiederverwendung des Katalysators und Extraktion) | Beispiel C-4a (erste Hydrierung) | Beispiel C-4b (Wiederverwendung des Katalysators und Extraktion) |
|---|---|---|---|---|
| Tertiäres Amin | 37.5 g Trihexylamin | Oberphase aus C-3a auf 37.5 g mit frischem Trihexylamin ergänzt | 37.5 g Trihexylamin | Oberphase aus C-4a auf 37.5 g mit frischem Trihexylamin ergänzt |
| Polares Lösungsmittel (einqesetzt) | 12.0 g Methanol | 12.0 g Methanol, | 12.0 g Methanol<br>0.5 g Wasser | 12.0 g Methanol<br>0.5 g Wasser |
| Katalysator | 0.16 g $[Ru(PnOctyl_3)_4(H)_2]$, 0.08 g 1,2-Bis(dicyclohexylphosphino)ethan | Oberphase aus C-3a | 0.16 g $[Ru(PnOctyl_3)_4(H)_2]$, 0.08 g 1,2-Bis(dicyclohexylphosphino)ethan | Oberphase aus C-4a |
| Aufpressen von $CO_2$ | Auf 1.8 MPa abs | Auf 1.6 MPa abs | Auf 1.8 MPa abs | Auf 1.7 MPa abs |
| Aufpressen von $H_2$ | Auf 8.0 MPa abs | Auf 8.0 MPa | Auf 8.0 MPa abs | Auf 8.0 MPa abs |
| Erwärmen | 70°C | 70°C | 70°C | 70°C |
| Reaktionszeit | 16 Stunden | 1.5 Stunden | 16 Stunden | 1.5 Stunden |
| Wasserzugabe nach der Reaktion | 1.0 g | 1.0 g | 1.0 g | 1.0 g |
| Oberphase | 19.9 g | 24.7 g | 19.7 g | 24.0 g |
| Unterphase | 30.8 g<br>6.8 % Ameisensäure | 24.4 g<br>6.0 % Ameisensäure | 31.1 g<br>7.1 % Ameisensäure | 26.8 g<br>6.4 % Ameisensäure |
| $c_{Ru}$ Oberphase nach Reaktion und Wasserzugabe | 205 ppm | 135 ppm | 250 ppm | 175 ppm |
| $c_{Ru}$ Unterphase nach Reaktion und Wasserzugabe | 145 ppm | - | 125 ppm | - |
| $c_{Ru}$ Unterphase nach Extraktion | - | 4 ppm | - | 4 ppm |

**Tabelle 1.10**

| | Beispiel C-5a (erste Hydrierung) | Beispiel C-5b (Wiederverwendung des Katalysators und Extraktion) | Beispiel C-6a (erste Hydrierung) | Beispiel C-6b (Wiederverwendung des Katalysators und Extraktion) |
|---|---|---|---|---|
| Tertiäres Amin | 37.5 g Trihexylamin | Oberphase aus C-5a auf 37.5 g mit frischem Trihexylamin erqänzt | 37.5 g Trihexylamin | Oberphase aus C-6a auf 37.5 g mit frischem Trihexylamin ergänzt |
| Polares Lösungsmittel (eingesetzt) | 12.0 g Methanol | 12.0 g Methanol | 12.0 g Methanol | |
| Katalysator | 0.16 g $[Ru(PnOctyl_3)_4(H)_2]$ | Oberphase aus C-5a | 0.16 g $[Ru(PnOctyl_3)_4(H)_2]$ | Oberphase aus C-6a |
| Aufpressen von $CO_2$ | Auf 1.7 MPa abs | Auf 1.6 MPa abs | Auf 1.7 MPa abs | Auf 1.7 MPa abs |
| Aufpressen von $H_2$ | Auf 8.0 MPa abs | Auf 8.0 MPa | Auf 8.0 MPa abs | Auf 8.0 MPa abs |
| Erwärmen | 50°C | 50°C | 50°C | 50°C |
| Reaktionszeit | 1.5 Stunden | 1.5 Stunden | 1.5 Stunden | 1.5 Stunden |
| Wasserzugabe nach der Reaktion | 1.0 g | 1.0 g | 2.5 g | 1.0 g |
| Oberphase | 22.1 g | 27.1 g | 23.8 g | 28.6 g |
| Unterphase | 27.8 g<br>6.7 % Ameisensäure | 22.1 g<br>4.5 % Ameisensäure | 26.9 g<br>6.2 % Ameisensäure | 20.6 g<br>4.8 % Ameisensäure |
| $c_{Ru}$ Oberphase nach Reaktion und Wasserzugabe | 420 ppm | 310 ppm | 400 ppm | 310 ppm |
| $c_{Ru}$ Unterphase nach Reaktion und Wasserzugabe | 14 ppm | - | 4 ppm | - |
| $c_{Ru}$ Unterphase nach Extraktion | - | 2 ppm | - | 2 ppm |

**Tabelle 1.11**

| | Beispiel C-7a (erste Hydrierung) | Beispiel C-7b (Wiederverwendung des Katalysators und Extraktion) |
|---|---|---|
| Tertiäres Amin | 37.5 g Trihexylamin | Oberphase aus C-7a auf 37.5 g mit frischem Trihexylamin ergänzt |
| Polares Lösungsmittel (eingesetzt) | 12.0 g Methanol<br>0.25 g Wasser | 12.0 g Methanol<br>0.25 g Wasser |
| Katalysator | 0.16 g $[Ru(PnOctyl_3)_4(H)_2]$, ], 0.08 g 1,2-Bis(dicyclohexylphosphino)ethan | Oberphase aus C-7a |
| Aufpressen von $CO_2$ | Auf 1.5 MPa abs | Auf 1.6 MPa abs |

(fortgesetzt)

| | Beispiel C-7a (erste Hydrierung) | Beispiel C-7b (Wiederverwendung des Katalysators und Extraktion) |
|---|---|---|
| Aufpressen von $H_2$ | Auf 8.0 MPa abs | Auf 8.0 MPa |
| Erwärmen | 50°C | 50°C |
| Reaktionszeit | 1.5 Stunden | 1.5 Stunden |
| Wasserzugabe nach der Reaktion | 1.0 g | 1.0 g |
| Oberphase | 17.5 g | 19.7 g |
| Unterphase | 33.0 g<br>7.3 % Ameisensäure | 30.6 g<br>7.1 % Ameisensäure |
| $c_{Ru}$ Oberphase nach Reaktion und Wasserzugabe | 370 ppm | 260 ppm |
| $c_{Ru}$ Unterphase nach Reaktion und Wasserzugabe | 34 ppm | - |
| $c_{Ru}$ Unterphase nach Extraktion | - | 16 ppm |

**Tabelle 1.12**

| | Beispiel C-8a (erste Hydrierung) | Beispiel C-8b (Wiederverwendung des Katalysators und Extraktion) | Beispiel C-8c (Wiederverwendung des Katalysators und Extraktion) |
|---|---|---|---|
| Tertiäres Amin | 37.5 g Trihexylamin | Oberphase aus C-8a auf 37.5 g mit frischem Trihexylamin ergänzt | Oberphase aus C-8b auf 37.5 g mit frischem Trihexylamin ergänzt |
| Polares Lösungsmittel (eingesetzt) | 12.0 g Methanol | 12.0 g Methanol | 12.0 g Methanol |
| Katalysator | 0.16 g [Ru(PnOctyl$_3$)$_4$(H)$_2$], ], 0.08 g 1,2-Bis(dicyclohexylphosphino)ethan | Oberphase aus C-8a | Oberphase aus C-8b |
| Aufpressen von $CO_2$ | Auf 1.7 MPa abs | Auf 1.8 MPa abs | Auf 1.6 MPa abs |
| Aufpressen von $H_2$ | Auf 8.0 MPa abs | Auf 8.0 MPa | Auf 8.0 MPa |
| Erwärmen | 70°C | 70°C | 70°C |
| Reaktionszeit | 16 Stunden | 1.5 Stunden | 1.5 Stunden |
| Wasserzugabe nach der Reaktion | 1.0 g | 1.0 g | 1.0 g |
| Oberphase | 20.4 g | 27.3 g | 23.7 g |
| Unterphase | 29.8 g<br>6.7 % Ameisensäure | 22.3 g<br>5.7 % Ameisensäure | 25.3 g<br>4.9 % Ameisensäure |

(fortgesetzt)

| | Beispiel C-8a (erste Hydrierung) | Beispiel C-8b (Wiederverwendung des Katalysators und Extraktion) | Beispiel C-8c (Wiederverwendung des Katalysators und Extraktion) |
|---|---|---|---|
| $c_{Ru}$ Oberphase nach Reaktion und Wasserzugabe | 215 ppm | 150 ppm | 110 ppm |
| $c_{Ru}$ Unterphase nach Reaktion und Wasserzugabe | 145 ppm | 14 ppm | - |
| $c_{Ru}$ Unterphase nach Extraktion | - | - | 3 ppm |

**Tabelle 1.13**

| | Beispiel C-9a (erste Hydrierung) | Beispiel C-9b (Wiederverwendung des Katalysators und Extraktion) | Beispiel C-9c (Wiederverwendung des Katalysators und Extraktion) |
|---|---|---|---|
| Tertiäres Amin | 37.5 g Trihexylamin | Oberphase aus C-9a auf 37.5 g mit frischem Trihexylamin ergänzt | Oberphase aus C-9b auf 37.5 g mit frischem Trihexylamin ergänzt |
| Polares Lösungsmittel (eingesetzt) | 12.0 g Methanol<br>0.5 g Wasser | 12.0 g Methanol | 12.0 g Methanol |
| Katalysator | 0.16 g $[Ru(PnOctyl_3)_4(H)_1]$, ], 0.08 g 1,2-Bis(dicyclohexylphosphino)ethan | Oberphase aus C-9a | Oberphase aus C-9b |
| Aufpressen von $CO_2$ | Auf 1.5 MPa abs | Auf 1.6 MPa abs | Auf 1.6 MPa abs |
| Aufpressen von $H_2$ | Auf 8.0 MPa abs | Auf 8.0 MPa | Auf 8.0 MPa |
| Erwärmen | 70°C | 70°C | 70°C |
| Reaktionszeit | 16 Stunden | 1.5 Stunden | 1.5 Stunden |
| Wasserzugabe nach der Reaktion | 1.0 g | 1.0 g | 1.0 g |
| Oberphase | 19.7 g | 27.8 g | 25.6 g |
| Unterphase | 31.6 g<br>7.0 % Ämeisensäure | 22.6 g<br>6.1 % Ameisensäure | 24.4 g<br>6.1 % Ameisensäure |
| $c_{Ru}$ Oberphase nach Reaktion und Wasserzugabe | 235 ppm | 155 ppm | 125 ppm |
| $c_{Ru}$ Unterphase nach Reaktion und Wasserzugabe | 110 ppm | 11 ppm | - |
| $c_{Ru}$ Unterphase nach Extraktion | - | - | 3 ppm |

Beispiele D1-D4 (Thermische Abtrennung des polaren Lösungsmittels von den Trialkylamin-Solvent-Ameisensäuregemischen, welche nach der Extraktion als Produktphase vorliegen.)

Alkohol und Wasser werden von der Produktphase (enthält das Ameisensäure/Amin-Addukt) unter vermindertem Druck mittels eines Rotationsverdampfers abdestilliert. Im Sumpf entsteht ein zweiphasiges Gemisch (Trialkylamin- und Ameisensäure/Amin-Addukt-Phase), die beiden Phasen werden getrennt und der Ameisensäuregehalt der Unterphase wurde durch Titration mit 0,1 N KOH in MeOH potentiometrisch mit einem "Mettler Toledo DL50"-Titrator bestimmt. Der Amin- und Alkoholgehalt wird durch Gaschromatographie bestimmt. Die Parameter und Ergebnisse der einzelnen Versuche sind in Tabelle 1.14 wiedergegeben.

Die Beispiele D-1 bis D-4 zeigen, sich dass beim erfindungsgemäßen Verfahren verschiedene polare Lösungsmittel unter milden Bedingungen von der Produktphase abtrennen lassen, wobei eine ameisensäurereichere Unterphase sowie eine Oberphase, bestehend überwiegend aus tertiärem Amin, anfällt.

**Tabelle 1.14**

| | Beispiel D-1 | Beispiel D-2 | Beispiel D-3 | Beispiel D-4 |
|---|---|---|---|---|
| Einsatzgemisch (Gew.%) | 18.7 g<br>7.2 % Ameisensäure<br>26.4 % 1-Propanol<br>15.5 % Wasser<br>48.3 % Trihexylamin | 19.3 g<br>5.8 % Ameisensäure<br>22.8 % 2-Propanol<br>4.1 % Wasser<br>67.2 % Trihexylamin | 81.8 g<br>7.3 % Ameisensäure<br>41.3 % Methanol<br>15.4 % Wasser<br>35.9 % Tripentylamin | 88.6 g<br>9.2 % Ameisensäure<br>31.4 % Ethanol<br>11.3 % Wasser<br>48.1 % Tripentylamin |
| Ameisensäure : Amin Einsatzqemisch | 1 : 1.2 | 1 : 2.0 | 1:1 | 1:1.1 |
| Druck | 20 mbar | 20 mbar | 200 mbar | 200 mbar |
| Temperatur | 50°C | 50°C | 100°C | 110°C |
| Ameisensäuregehalt Unterphase nach Destillation (Gew.%) | 16.4 % | 18.0 % | 23.7 % | 22.7 % |
| Ameisensäure : Amin Unterphase nach Destillation (molares Verhältnis) | 1 : 0.76 | 1 : 0.78 | 1 : 0.6 | 1 : 0.56 |
| Wiederfindungsrate Ameisensäure nach Destillation | 95.3 % | 93.7 % | 90.4 % | 95.2% |

Beispiele E1 und E2 (Thermische Abtrennung des polaren Lösungsmittels von den Trialkylamin-Solvent-Ameisensäuregemischen und Spaltung des Ameisensäure-Amin-Adduktes)

Alkohol und Wasser werden von der Produktphase (enthält das Ameisensäure/Amin-Addukt) unter vermindertem Druck mittels eines Rotationsverdampfers abdestilliert. Im Sumpf entsteht ein zweiphasiges Gemisch (Trialkylamin- und Ameisensäure/Amin-Addukt-Phase) und die beiden Phasen werden getrennt. Die Zusammensetzung des Destillates (enthaltend den Großteil des Methanols sowie des Wassers), der Oberphase (enthaltend das freie Trialkylamin) und der Unterphase (enthaltend das Ameisensäure-Amin-Addukt) wurde durch Gaschromatographie und durch Titration der Ameisensäure gegen 0,1 N KOH in MeOH potentiometrisch mit einem "Mettler Toledo DL50"-Titrator bestimmt. Die Ameisensäure wird dann aus der Unterphase aus dem ersten Schritt in einer Vakuumdestillationsapparatur über eine 10 cm Vigreuxkolonne thermisch vom Trialkylamin abgespalten. Dabei wird nach vollständiger Abspaltung der Ameisensäure ein einphasiger Sumpf, bestehend aus dem reinen Trialkylamin erhalten, welches zur Extraktion des Katalysators und Rückführung in die Hydrierung eingesetzt werden kann. Im Destillat findet sich die Ameisensäure, sowie Restwasser. Die Zusammensetzung des Sumpfes sowie des Destillates wurde durch Gaschromatographie und durch Titration der Ameisensäure mit gegen 0,1 N KOH in MeOH potentiometrisch mit einem "Mettler Toledo DL50"-Titrator bestimmt. Die Parameter und Ergebnisse der einzelnen Versuche sind in Tabelle 1.15 wiedergegeben.

Die Beispiele E-1 und E2 zeigen, sich dass beim erfindungsgemäßen Verfahren verschiedene polare Lösungsmittel unter milden Bedingungen von der Produktphase abtrennen lassen, wobei eine ameisensäurereichere Unterphase sowie eine Oberphase, bestehend überwiegend aus tertiärem Amin. Von dieser ameisensäurereichen Unterphase kann

dann die Ameisensäure bei höheren Temperaturen vom Trialkylamin abgespalten werden, wobei das freie Trialklyamin anfällt. Die so gewonnene Ameisensäure enthält noch etwas Wasser, welches aber durch eine Kolonne mit größerer Trennleistung von der Ameisensäure abgetrennt werden kann. Das sowohl bei der Abtrennung des Lösungsmittels als auch bei der thermischen Spaltung anfallende Trialkylamin kann dazu verwendet werden, den Katalysator aus dem Produktstrom abzureichern.

**Tabelle 1.15**

| | Beispiel D-1a (Abtrennung des polaren Solvents) | Beispiel D-1b (Spaltung des Ameisensäure-Amin-Adduktes) | Beispiel D-2a (Abtrennung des polaren Solvents | Beispiel D-2b (Spaltung des Ameisensäure-Amin-Adduktes) |
|---|---|---|---|---|
| Einsatzgemisch (Gew.%) | 199.8 g<br>8.9 % Ameisensäure<br>28.4 % Methanol<br>5.6 % Wasser<br>57.1 % Trihexylamin | Unterphase aus D1-a | 199.8 g<br>7.8 % Ameisensäure<br>33.0 % Methanol<br>15.1 % Wasser<br>44.0 % Trihexylamin | Unterphase aus D2-a |
| Ameisensäure : Amin Einsatzgemisch | 1 : 1.1 | 1 : 0.64 | 1 : 1 | 1 : 0.89 |
| Druck | 200 mbar | 90 mbar | 200 mbar | 90 mbar |
| Temperatur | 120°C | 153°C | 120°C | 153°C |
| Unterphase im Sumpf nach Destillation (Gew.%) | 79.8 g<br>22.1 % Ameisensäure<br>1.5 % Wasser<br>76.4 % Trihexylamin | 63.6 g<br>100 % Trihexylamin | 69.4 g<br>14.9 % Ameisensäure<br>6.9 % Wasser<br>78.2 % Trihexylamin | 55.5 g<br>99.7 % Trihexylamin<br>0.3 % Wasser |
| Oberphase im Sumpf nach Destillation | 50.5 g<br>100 % Trihexylamin | einphasig | 32.7 g<br>99.7 % Trihexylamin<br>0.3 % Wasser | einphasig |
| Destillat | 66.6 g<br>0.3 % Ameisensäure<br>81.2 % Methanol<br>18.5 % Wasser | 14.9 g<br>92.1 % Ameisensäure<br>7.9 % Wasser | 93.1 g<br>70.1 % Methanol<br>29.9 % Wasser | 12.9 g<br>85.0 % Ameisensäure<br>15 % Wasser |

Die Zeichnungen zeigen im Einzelnen:

| | |
|---|---|
| Figur 1 | ein Blockdiagramm einer bevorzugten Ausführungsform des erfindungsgemäßen Verfahrens, |
| Figuren 2A, 2B und2C | jeweils unterschiedliche, bevorzugte Varianten für die fraktionierte Abtrennung von polarem Lösungsmittel und Wasser, und |
| Figur 3 | ein Blockdiagramm einer weiteren bevorzugten Ausführungsform des erfindungsgemäßen Verfahrens. |

Bei der Ausführungsform gemäß Figur 1 werden Kohlendioxid, Strom 1, und Wasserstoff, Strom 2, in den Hydrierreaktor I geführt. Darin werden diese in Gegenwart eines Katalysators enthaltend ein Element aus der 8., 9. oder 10. Gruppe des Periodensystems, eines tertiären Amins und eines polaren Lösungsmittels zu Ameisensäure-Amin-Addukten umgesetzt.

Der Austrag aus dem Hydrierreaktor I (Strom 3), der ein- oder zweiphasig sein kann, wird mit dem überwiegend Wasser enthaltenden Strom 16 aus dem Destillationsgefäß IV versetzt.

Es ist auch möglich, den überwiegend Wasser enthaltenden Strom 16 unmittelbar in das Phasentrenngefäß II zuzugeben. Im Phasentrenngefäß II liegt eine Unterphase vor, die mit Ameisensäure-Amin-Addukten sowie dem polaren Lösungsmittel angereichert ist, und eine Oberphase, die mit dem tertiären Amin, und im Falle des Einsatzes eines homogenen Katalysators, auch mit diesem angereichert ist. Die Oberphase 4 wird zum Hydrierreaktor 1 zurückgeführt. Die Unterphase 5 wird einer Extraktionsapparatur III zugeführt, worin Katalysatorreste mit dem tertiären Amin aus dem Phasentrenngefäß V (Strom 13) extrahiert werden. Das tertiäre Amin mit den Katalysatorresten (Strom 6) aus der Extraktionseinheit IIII wird in den Hydrierreaktor I zurückgeführt. Die Produktphase 7 aus der Extraktionseinheit III wird der thermischen Abtrenneinheit IV zugeführt, um das polare Lösungsmittel sowie das Wasser thermisch von den Ameisensäure-Amin-Addukten abzutrennen. Der Strom 7 kann dabei noch zuvor über ein Adsorberbett geführt werden, um letzte Katalysatorspuren aus diesem Strom zu entfernen. Das polare Lösungsmittel, das in der Destillationseinheit IV thermisch abgetrennt wird, wird in den Hydrierreaktor I, als Strom 8, zurückgeführt, das Wasser wird als Strom 16 zum Austrag aus dem Hydrierreaktor (Strom 3) zurückgeführt, und das zweiphasige Sumpfgemisch aus der Destillationseinheit IV, enthaltend die Ameisensäure-Amin-Addukte und das tertiäre Amin, wird dem Phasentrenngefäß V zugeführt. Die Ameisensäure-Amin-Addukte werden im Phasentrenngefäß V abgetrennt und der Destillationseinheit VI als Strom 10 zugeführt, worin diese in freie Ameisensäure und tertiäres Amin thermisch gespalten werden. Die freie Ameisensäure wird als Kopfprodukt, Strom 12, abgezogen und der zweiphasige Sumpf aus der Destillationseinheit VI, enthaltend tertiäres Amin und nicht gespaltene Ameisensäure/Amin-Addukte, Strom 11, wird erneut dem Phasentrenngefäß V zugeführt. Das tertiäre Amin, welches im Phasentrenngefäß V abgetrennt wird, wird als Strom 13 der Extraktionseinheit III zugeführt, um Katalysatorreste zu extrahieren extrahieren bzw. Teile von Strom 13 können direkt in den Hydrierreaktor I zurückgeführt werden, falls nicht alles Trialkylamin zur Extraktion benötigt wird.

Die Figuren 2A, 2B und 2C zeigen schematisch jeweils unterschiedliche Varianten für die thermische Abtrennung von Wasser und polarem Lösungsmittel.

Hierbei zeigt Figur 2A die Abtrennung beider Ströme, d.h. des überwiegend polares Lösungsmittel enthaltenden Stromes 8 und des überwiegend Wasser enthaltenden Stromes 16 in einer einzigen Destillationskolonne, die eine Kolonne mit Seitenabzug auch eine Trennwandkolonne sein kann.

Figur 2B zeigt schematisch eine Ausführungsform mit einer 2-Kolonnen-Variante (Kolonnen IVa und IVb), wobei in der ersten Kolonne, IVa, die leichter siedende Komponente, in der Regel das polare Lösungsmittel, und in der zweiten Kolonne, IVb, der ein Eduktstrom 7a zugeführt wird, der von polarem Lösungsmittel abgereichert ist, die mittelsiedende Komponente, in der Regel das Wasser, als Strom 16, abgetrennt wird.

Figur 2C zeigt eine weitere Ausführungsform für die Abtrennung von Wasser und polarem Lösungsmittel, wobei in einer ersten Destillationskolonne IVc zunächst die Phase 9 abgetrennt wird, enthaltend die Ameisensäure/Amin-Addukte sowie das tertiäre Amin, und anschließend ein Strom 7b in einer zweiten Destillationskolonne IVd aufgetrennt wird, unter Erhalt eines überwiegend polares Lösungsmittel enthaltenden Stromes 8 und eines überwiegend Wasser enthaltenden Stromes 16.

Figur 3 zeigt das Blockdiagramm einer weiteren bevorzugten Ausführungsform des erfindungsgemäßen Verfahrens, wobei, abweichend von der in Figur 1 dargestellten Ausführungsform, die überwiegend Wasser enthaltende Fraktion, Strom 16, aus der Destillationseinheit IV ausgeschleust und in den Austrag aus dem Hydrierreaktor I, Strom 3, frisches Wasser, Strom 17, zugeführt wird.

Darüber hinaus unterscheidet sich die in Figur 3 schematisch dargestellte Ausführungsform gegenüber der in Figur 1 dargestellten Variante in der Weise, dass ein weiteres Phasentrenngefäß, VII, vorgesehen ist, dem der Produktstrom 9 aus der Destillationseinheit IV, enthaltend die Ameisensäure/Amin-Addukte und das tertiäre Amin, zugeführt wird, und worin das tertiäre Amin, als Strom 15, abgetrennt und in die Extraktionseinheit III, als Strom 15, recycliert wird, bzw. Teile von Strom 15 direkt in den Hydrierreaktor I zurückgeführt werden, falls nicht alles Trialkylamin zur Extraktion benötigt wird.

Der Ameisensäure/Amin-Addukte enthaltende Strom 14 wird anschließend der Destillationseinheit VI zugeführt, worin diese in freie Ameisensäure, Strom 12, gespalten werden. Der zweiphasige Sumpf aus der Destillationseinheit VI, enthaltend tertiäres Amin und nicht gespaltene Ameisensäure/Amin-Addukte wird dem Phasentrenngefäß V zugeführt, worin ein Strom 13 abgetrennt wird, enthaltend das tertiäre Amin, der in die Destillationseinheit III recycliert wird, um Katalysatorreste zu extrahieren. Nicht gespaltene Ameisensäure/Amin-Addukte, Strom 10, werden in die Destillationseinheit VI recycliert.

## Patentansprüche

1. Verfahren zur Herstellung von Ameisensäure durch Umsetzung von Kohlendioxid (1) mit Wasserstoff (2) in einem Hydrierreaktor (I) in Gegenwart

    - eines Katalysators, enthaltend ein Element aus der 8., 9 oder 10. Gruppe des Periodensystems,

- eines tertiären Amins, enthaltend mindestens 12 Kohlenstoffatome pro Molekül, sowie
- eines polaren Lösungsmittels, enthaltend einen oder mehrere Monoalkohole, ausgewählt aus Methanol, Ethanol, Propanolen und Butanolen,

unter Bildung von Ameisensäure/Amin-Addukten als Intermediaten, die anschließend thermisch gespalten werden, wobei ein tertiäres Amin eingesetzt wird, das einen um mindestens 5 °C höheren Siedepunkt als Ameisensäure aufweist, und wobei
sich bei der Umsetzung im Hydrierreaktor (I) ein Reaktionsgemisch bildet, enthaltend das polare Lösungsmittel, die Ameisensäure/Amin-Addukte, das tertiäre Amin und den Katalysator, das aus dem Reaktor ausgetragen wird,
**dadurch gekennzeichnet, dass**
die Aufarbeitung des Austrags (3) aus dem Hydrierreaktor (I) nach den folgenden Verfahrensschritten durchgeführt wird:

1) Zugabe von Wasser zum Austrag (3) aus dem Hydrierreaktor, wobei man zwei Flüssigphasen erhält,
2) Auftrennung der zwei Flüssigphasen in einem Phasentrenngefäß (II), Rückführung der Oberphase (4) aus dem Phasentrenngefäß (II) in den Hydrierreaktor (I) und Weiterleitung der Unterphase (5) aus dem Phasentrenngefäß (II) in eine Extraktionsapparatur (III), worin
3) Reste an Katalysator mit demselben tertiären Amin, das in der Hydrierung eingesetzt wurde, extrahiert, das mit Katalysator beladene tertiäre Amin (6) in den Hydrierreaktor (I) recycliert wird, und der katalysatorfreie Strom von mit den Ameisensäure/Amin-Addukten beladenem polaren Lösungsmittel (7) in eine Destillationseinheit (IV) weitergeleitet wird, worin
4) eine fraktionierte Destillation durchgeführt wird unter Erhalt einer ersten Fraktion (8), die überwiegend das polare Lösungsmittel enthält, und die in den Hydrierreaktor (I) recycliert wird, einer zweiten Fraktion (16), die überwiegend Wasser enthält, und die in den Austrag (3) aus dem Hydrierreaktor (I) recycliert oder verworfen wird, sowie einer Fraktion (9),
5) in einem Phasentrenngefäß (V) in eine Oberphase enthaltend überwiegend das tertiäre Amin und eine Unterphase, enthaltend überwiegend die Ameisensäure/Amin-Addukte, aufgetrennt wird, wobei
6) die Unterphase aus dem Phasentrenngefäß (V) einer thermischen Spalteinheit (VI) zugeführt, und darin in einen das tertiäre Amin enthaltenden Strom (11), der in das Phasentrenngefäß (V) zurückgeführt wird und in reine Ameisensäure (12) gespalten wird, und wobei

ein das tertiäre Amin enthaltender Strom (13) aus dem Phasentrenngefäß (V) in die Extraktionsapparatur (III) als selektives Lösungsmittel für den Katalysator geleitet wird.

2. Verfahren nach Anspruch 1, **dadurch gekennzeichnet, dass** im Verfahrensschritt (6) lediglich ein Teilstrom des das tertiäre Amin enthaltenden Stromes (13) aus dem Phasentrenngefäß (V) in die Extraktionsapparatur (III) als selektives Lösungsmittel für den Katalysator und der verbleibende Anteil des das tertiäre Amin enthaltenden Stroms (13) in den Hydrierreaktor (I) geleitet wird.

3. Verfahren nach Anspruch 1 oder 2, **dadurch gekennzeichnet, dass** man als tertiäres Amin ein Amin der allgemeinen Formel (Ia)

$$NR^1R^2R^3 \qquad (Ia)$$

einsetzt, in der die Reste $R^1$ bis $R^3$ gleich oder verschieden sind und abhängig voneinander einen unverzweigten oder verzweigten, acyclischen oder cyclischen, aliphatischen, araliphatischen oder aromatischen Rest mit jeweils 1 bis 6 Kohlenstoffatomen darstellen, wobei einzelne Kohlenstoffatome unabhängig voneinander auch durch eine Heterogruppe ausgewählt aus der Gruppe -O- und >N- substituiert sein können sowie zwei oder alle drei Reste unter Bildung einer mindestens jeweils vier Atome umfassenden Kette auch miteinander verbunden sein können, mit der Bedingung, dass das tertiäre Amin mindestens 12 Kohlenstoffatome pro Molekül enthält.

4. Verfahren nach Anspruch 3, **dadurch gekennzeichnet, dass** man als tertiäres Amin ein Amin der allgemeinen Formel (Ia) einsetzt, in der die Reste $R^1$ bis $R^3$ unabhängig voneinander ausgewählt sind aus der Gruppe $C_1$- bis $C_{12}$-Alkyl, $C_5$-bis $C_8$-Cycloalkyl, Benzyl und Phenyl.

5. Verfahren nach Anspruch 3, **dadurch gekennzeichnet, dass** man als tertiäres Amin ein gesättigtes Amin der allgemeinen Formel (Ia) einsetzt.

**6.** Verfahren nach Anspruch 3, **dadurch gekennzeichnet, dass** man als tertiäres Amin ein Amin der allgemeinen Formel (Ia) einsetzt, in der die Reste $R^1$ bis $R^3$ unabhängig voneinander ausgewählt sind aus $C_5$- und $C_6$-Alkyl.

**7.** Verfahren nach einem der Ansprüche 1 bis 6, **dadurch gekennzeichnet, dass** man als polares Lösungsmittel Methanol und/oder Ethanol einsetzt.

**8.** Verfahren nach einem der Ansprüche 1 bis 7, **dadurch gekennzeichnet, dass** die in Verfahrensschritt 1) zugegebene Wassermenge so bemessen wird, dass der Wassergehalt in der Unterphase (5) aus dem Phasentrenngefäß (II), bezogen auf das Gesamtgewicht der Unterphase (5) aus dem Phasentrenngefäß (II) 0,1 bis 50 Gew.-%, bevorzugt 2 bis 30 Gew.-%, beträgt.

**9.** Verfahren nach einem der Ansprüche 1 bis 8, **dadurch gekennzeichnet, dass** der Katalysator ein homogener Katalysator ist.

**10.** Verfahren nach Anspruch 9, **dadurch gekennzeichnet, dass** der homogene Katalysator eine metallorganische Komplexverbindung ist, enthaltend ein Element aus der 8., 9. oder 10. Gruppe des Periodensystems und mindestens eine Phosphingruppe mit mindestens einem unverzweigten oder verzweigten, acyclischen oder cyclischen aliphatischen Rest mit 1 bis 12 Kohlenstoffatomen, wobei einzelne Kohlenstoffatome auch durch >P- substituiert sein können.

**11.** Verfahren nach einem der Ansprüche 1 bis 10, **dadurch gekennzeichnet, dass** die Umsetzung im Hydrierreaktor (I) bei einer Temperatur im Bereich zwischen 20 und 200 °C und bei einem Druck im Bereich zwischen 0,2 und 30 MPa abs durchgeführt wird.

**12.** Verfahren nach Anspruch 11, **dadurch gekennzeichnet, dass** der Druck im Hydrierreaktor (I) und im Phasentrenngefäß (II) gleich oder annähernd gleich ist.

**13.** Verfahren nach Anspruch 11, **dadurch gekennzeichnet, dass** die Temperatur im Hydrierreaktor (I) und im Phasentrenngefäß (II) gleich oder annähernd gleich ist.

**Claims**

**1.** A process for preparing formic acid by reaction of carbon dioxide (1) with hydrogen (2) in a hydrogenation reactor (I) in the presence of

- a catalyst comprising an element of group 8, 9 or 10 of the Periodic Table,
- a tertiary amine comprising at least 12 carbon atoms per molecule and
- a polar solvent comprising one or more monoalcohols selected from among methanol, ethanol, propanols and butanols,

to form formic acid/amine adducts as intermediates which are subsequently thermally dissociated, where a tertiary amine which has a boiling point at least 5°C higher than that of formic acid is used and
a reaction mixture comprising the polar solvent, the formic acid/amine adducts, the tertiary amine and the catalyst carried from the reactor is formed in the reaction in the hydrogenation reactor (I),
wherein
the work-up of the output (3) from the hydrogenation reactor (I) is carried out by the following process steps:

1) addition of water to the output (3) from the hydrogenation reactor to give two liquid phases,
2) separation of the two liquid phases in a phase separation vessel (II), recirculation of the upper phase (4) from the phase separation vessel (II) to the hydrogenation reactor (I) and passing-on of the lower phase (5) from the phase separation vessel (II) to an extraction apparatus (III) in which
3) residues of catalyst are extracted with the same tertiary amine which was used in the hydrogenation, the catalyst-laden tertiary amine (6) is recycled to the hydrogenation reactor (I) and the catalyst-free stream of polar solvent (7) loaded with the formic acid/amine adducts is passed on to a distillation unit (IV) in which
4) a fractional distillation is carried out to give a first fraction (8) which comprises predominantly the polar solvent and is recycled to the hydrogenation reactor (I), a second fraction (16) which comprises predominantly water and is recycled to the output (3) from the hydrogenation reactor (I) or is discarded and also a fraction (9),

5) is separated in a phase separation vessel (V) into an upper phase comprising predominantly the tertiary amine and a lower phase comprising predominantly the formic acid/amine adducts, where
6) the lower phase from the phase separation vessel (V) is fed to a thermal dissociation unit (VI) and dissociated therein into a stream (11) which comprises the tertiary amine and is recirculated to the phase separation vessel (V) and pure formic acid (12) and

a stream (13) comprising the tertiary amine is conveyed from the phase separation vessel (V) into the extraction apparatus (III) as selective solvent for the catalyst.

**2.** The process according to claim 1, wherein, in process step (6), only a substream of the stream (13) comprising the tertiary amine is conveyed from the phase separation vessel (V) into the extraction apparatus (III) as selective solvent for the catalyst and the remaining proportion of the stream (13) comprising the tertiary amine is conveyed into the hydrogenation reactor (I).

**3.** The process according to claim 1 or 2, wherein an amine of the general formula (Ia)

$$NR^1R^2R^3 \qquad (Ia)$$

where the radicals $R^1$ to $R^3$ are identical or different and are each, independently of one another, an unbranched or branched, acyclic or cyclic, aliphatic, araliphatic or aromatic radical having in each case from 1 to 6 carbon atoms, where individual carbon atoms can also be substituted, independently of one another, by a hetero group selected from the group consisting of -O- and >N- or two or all three radicals can also be joined to one another to form a chain comprising at least four atoms in each case, is used as tertiary amine, with the proviso that the tertiary amine comprises at least 12 carbon atoms per molecule.

**4.** The process according to claim 3, wherein an amine of the general formula (Ia) in which the radicals $R^1$ to $R^3$ are selected independently from the group consisting of $C_1$-$C_{12}$-alkyl, $C_5$-$C_8$-cycloalkyl, benzyl and phenyl is used as tertiary amine.

**5.** The process according to claim 3, wherein a saturated amine of the general formula (Ia) is used as tertiary amine.

**6.** The process according to claim 3, wherein an amine of the general formula (Ia) in which the radicals $R^1$ to $R^3$ are selected independently from $C_5$ and $C_6$-alkyl is used as tertiary amine.

**7.** The process according to any of claims 1 to 6, wherein methanol and/or ethanol are used as polar solvent.

**8.** The process according to any of claims 1 to 7, wherein the amount of water added in process step 1) is such that the water content in the lower phase (5) from the phase separation vessel (II), based on the total weight of the lower phase (5) from the phase separation vessel (II), is from 0.1 to 50% by weight, preferably from 2 to 30% by weight.

**9.** The process according to any of claims 1 to 8, wherein the catalyst is a homogeneous catalyst.

**10.** The process according to claim 9, wherein the homogeneous catalyst is a metal-organic complex comprising an element of group 8, 9 or 10 of the Periodic Table and at least one phosphine group having at least one unbranched or branched, acyclic or cyclic aliphatic radical having from 1 to 12 carbon atoms, with individual carbon atoms also being able to be substituted by > P-.

**11.** The process according to any of claims 1 to 10, wherein the reaction in the hydrogenation reactor (I) is carried out at a temperature in the range from 20 to 200°C and a pressure in the range from 0.2 to 30 MPa abs.

**12.** The process according to claim 11, wherein the pressure in the hydrogenation reactor (I) and in the phase separation vessel (II) is the same or approximately the same.

**13.** The process according to claim 11, wherein the temperature in the hydrogenation reactor (I) and in the phase separation vessel (II) is the same or approximately the same.

## Revendications

1. **1.** Procédé pour la production d'acide formique par mise en réaction de dioxyde de carbone (1) avec de l'hydrogène (2) dans un réacteur d'hydrogénation (I) en présence

   - d'un catalyseur, contenant un élément du 8e, 9e ou 10e groupe du système périodique,
   - d'une amine tertiaire, comportant au moins 12 atomes de carbone par molécule, ainsi que
   - d'un solvant polaire, contenant un ou plusieurs monoalcools, choisis parmi le méthanol, l'éthanol, les propanols et les butanols,

   avec formation d'adduits acide formique/amine en tant que composés intermédiaires, qui sont ensuite scindés par voie thermique,
   dans lequel on utilise une amine tertiaire qui présente un point d'ébullition supérieur d'au moins 5 °C à celui de l'acide formique, et dans lequel
   lors de la réaction dans le réacteur d'hydrogénation (I) il se forme un mélange réactionnel contenant le solvant polaire, les adduits acide formique/amine, l'amine tertiaire et le catalyseur, qui est déchargé du réacteur,
   **caractérisé en ce**
   **qu'**on effectue le traitement final du courant de décharge (3) provenant du réacteur d'hydrogénation (I) selon les étapes de processus suivantes :

   1) addition d'eau au courant de décharge (3) provenant du réacteur d'hydrogénation, de sorte qu'on obtient deux phases liquides,
   2) séparation des deux phases liquides dans un récipient de séparation de phases (II), recyclage de la phase supérieure (4) provenant du récipient de séparation de phases (II) dans le réacteur d'hydrogénation (I) et transfert de la phase inférieure (5) provenant du récipient de séparation de phases (II) dans un appareil d'extraction (III), dans lequel
   3) les résidus de catalyseur sont extraits avec la même amine tertiaire que celle ayant été utilisée dans l'hydrogénation, l'amine tertiaire (6) chargée de catalyseur est recyclée dans le réacteur d'hydrogénation (I), et le courant, privé de catalyseur, de solvant polaire (7) chargé des adduits acide formique/amine est transféré dans une unité de distillation (IV), dans laquelle
   4) est effectuée une distillation fractionnée avec obtention d'une première fraction (8) qui contient essentiellement le solvant polaire et qui est recyclée dans le réacteur d'hydrogénation (I), d'une deuxième fraction (16) qui contient essentiellement de l'eau et qui est rejetée ou recyclée dans le courant de décharge (3) provenant du réacteur (I), ainsi que d'une fraction (9), qui
   5) dans un récipient de séparation de phases (V) est fractionnée en une phase supérieure contenant principalement l'amine tertiaire et une phase inférieure, contenant principalement les adduits acide formique/amine,
   6) la phase inférieure provenant du récipient de séparation de phases (V) étant envoyée à une unité de coupure thermique (VI) et dans celle-ci scindée en un courant (11) contenant l'amine tertiaire, qui est renvoyé dans le récipient de séparation de phases (V) et est scindé en acide formique pur (12), et

   un courant (13) contenant l'amine tertiaire, provenant du récipient de séparation de phases (V) étant envoyé dans l'appareil d'extraction (III) en tant que solvant sélectif pour le catalyseur.

2. **2.** Procédé selon la revendication 1, **caractérisé en ce que** dans l'étape de processus (6) seul un courant partiel du courant (13) contenant l'amine tertiaire, provenant du récipient de séparation de phases (V) est envoyé dans l'appareil d'extraction (III) en tant que solvant sélectif pour le catalyseur et la partie restante du courant (13) contenant l'amine tertiaire est envoyée dans le réacteur d'hydrogénation (I).

3. **3.** Procédé selon la revendication 1 ou 2, **caractérisé en ce qu'**on utilise comme amine tertiaire une amine de formule générale (Ia)

   $$NR^1R^2R^3 \qquad (Ia)$$

   dans laquelle les radicaux $R^1$ à $R^3$ sont identiques ou différents et représentent chacun indépendamment un radical aliphatique acyclique ou cyclique, ramifié ou non ramifié, araliphatique ou aromatique, ayant chaque fois de 1 à 6 atomes de carbone, des atomes de carbone individuels pouvant, indépendamment les uns des autres, être également remplacés par un hétéroatome choisi dans le groupe constitué par -O- et >N- et deux ou tous les trois radicaux pouvant également être liés entre eux avec formation d'une chaîne comprenant au moins chaque fois quatre atomes,

étant entendu que l'amine tertiaire contient au moins 12 atomes de carbone par molécule.

4. Procédé selon la revendication 3, **caractérisé en ce qu'**on utilise comme amine tertiaire une amine de formule générale (Ia), dans laquelle les radicaux $R^1$ à $R^3$ sont choisis chacun indépendamment dans l'ensemble constitué par des groupes alkyle en $C_1$-$C_{12}$, cycloalkyle en $C_5$-$C_8$, benzyle et phényle.

5. Procédé selon la revendication 3, **caractérisé en ce qu'**on utilise comme amine tertiaire une amine saturée de formule générale (Ia).

6. Procédé selon la revendication 3, **caractérisé en ce qu'**on utilise comme amine tertiaire une amine de formule générale (Ia), dans laquelle les radicaux $R^1$ à $R^3$ sont choisis chacun indépendamment parmi des groupes alkyle en $C_5$ et $C_6$.

7. Procédé selon l'une quelconque des revendications 1 à 6, **caractérisé en ce qu'**on utilise comme solvant polaire le méthanol et/ou l'éthanol.

8. Procédé selon l'une quelconque des revendications 1 à 7, **caractérisé en ce qu'**on choisit la quantité d'eau ajoutée dans l'étape 1) du procédé de manière que la teneur en eau de la phase inférieure (5) provenant du récipient de séparation de phases (II) vaille de 0,1 à 50 % en poids, de préférence de 2 à 30 % en poids, par rapport au poids total de la phase inférieure (5) provenant du récipient de séparation de phases (II).

9. Procédé selon l'une quelconque des revendications 1 à 8, **caractérisé en ce que** le catalyseur est un catalyseur homogène.

10. Procédé selon la revendication 9, **caractérisé en ce que** le catalyseur homogène est un composé de type complexe organométallique, contenant un élément du 8$^e$, 9$^e$ ou 10$^e$ groupe du système périodique et au moins un groupe phosphine comportant au moins un radical aliphatique cyclique ou acyclique, ramifié ou non ramifié, ayant de 1 à 12 atomes de carbone, des atomes de carbone individuels pouvant également être remplacés par >P-.

11. Procédé selon l'une quelconque des revendications 1 à 10, **caractérisé en ce que** la réaction dans le réacteur d'hydrogénation (I) est effectuée à une température dans la plage comprise entre 20 et 200 °C et sous une pression dans la plage comprise entre 0,2 et 30 MPa (absolue).

12. Procédé selon la revendication 11, **caractérisé en ce que** la pression dans le réacteur d'hydrogénation (I) et dans le récipient de séparation de phases (II) est la même ou approximativement la même.

13. Procédé selon la revendication 11, **caractérisé en ce que** la température dans le réacteur d'hydrogénation (I) et dans le récipient de séparation de phases (II) est la même ou approximativement la même.

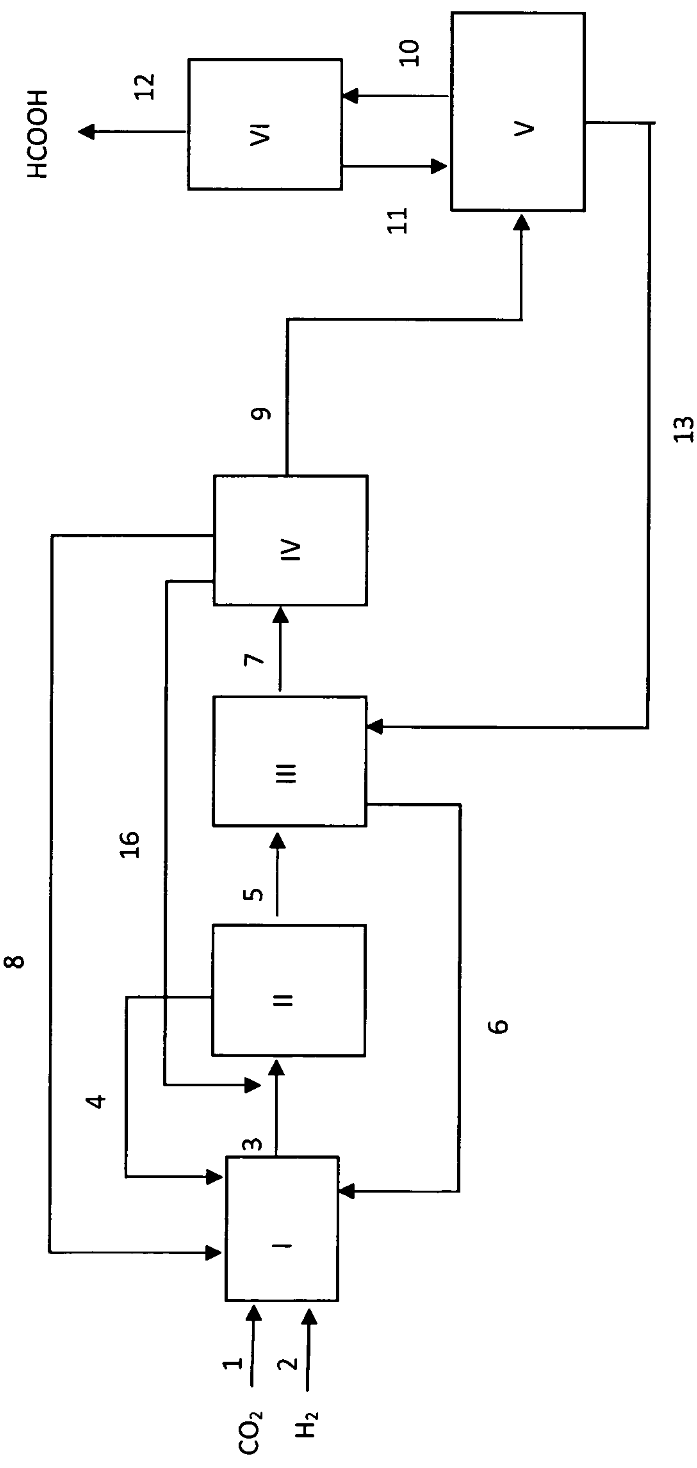


Figur 1

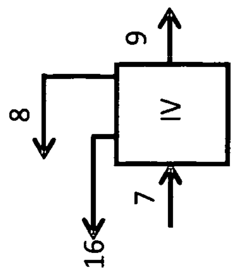


Figur 2A

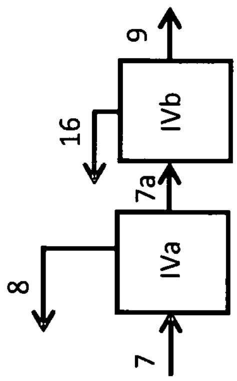


Figur 2B

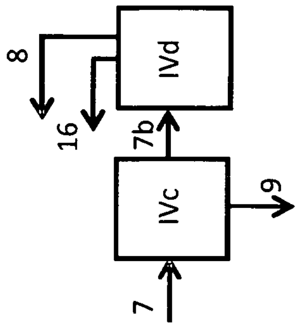


Figur 2C

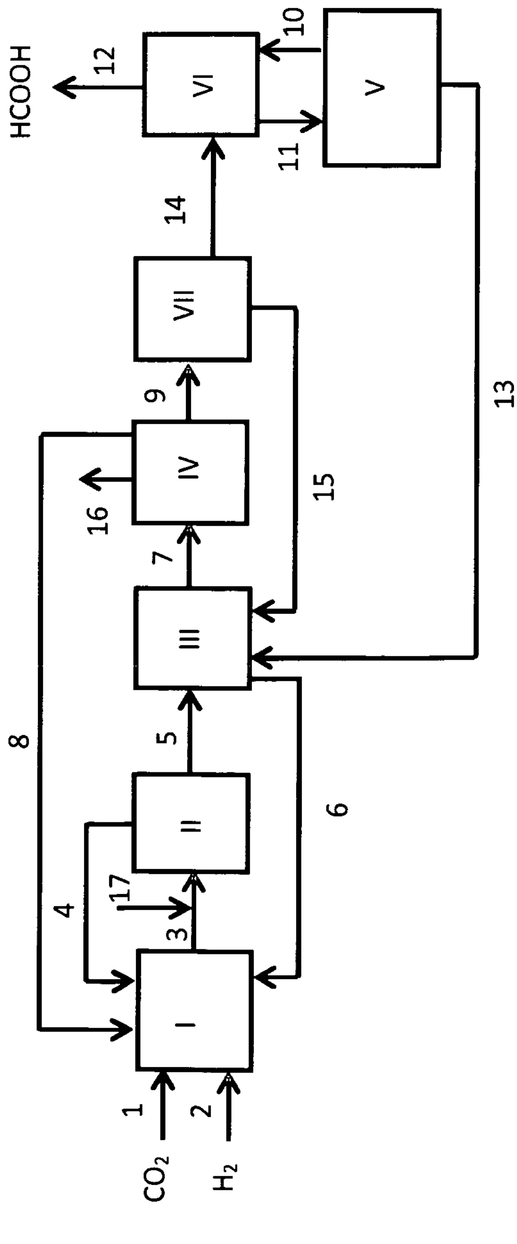


Figur 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 68920933
- WO 2008116799 A
- EP 0095321 A
- EP 0151510 A
- EP 0181078 A
- EP 0126524 A
- EP 0329337 A
- EP 0357243 A
- DE 4431233 A
- WO 2006021411 A
- EP 181078 B1
- EP 357243 B1


### In der Beschreibung aufgeführte Nicht-Patentliteratur


- **W. LEITNER.** *Angewandte Chemie,* 1995, vol. 107, 2391-2405
- **P. G. JESSOP ; T. IKARIYA ; R. NOYORI.** *Chemical Reviews,* 1995, vol. 95, 259-272
- **C. FELLAY ; N. YAN ; P.J. DYSON ; G. LAURENCZY.** *Chem. Eur. J.,* 2009, vol. 15, 3752-3760
- **C. FELLAY ; P.J. DYSON ; G. LAURENCZY.** *Angew. Chem.,* 2008, vol. 120, 4030-4032
- **B. LOGES ; A. BODDIEN ; H. JUNGE ; M. BELLER.** *Angew. Chem.,* 2008, vol. 120, 4026-4029
- **F. JOÓ.** *ChemSusChem,* 2008, vol. 1, 805-808
- **S. ENTHALER.** *ChemSusChem,* 2008, vol. 1, 801-804
- **S. FUKUZUMI ; T. KOBAYASHI ; T. SUENOBU.** *ChemSusChem,* 2008, vol. 1, 827-834
- **A. BODDIEN ; B. LOGES ; H. JUNGE ; M. BELLER.** *ChemSusChem,* 2008, vol. 1, 751-758
- **P.G. JESSOP ; T. IKARIYA ; R. NOYORI.** *Chem. Rev.,* 1995, vol. 95, 259-272
- **P.G. JESSOP ; F. JOÓ ; C.C. TAI.** *Coord. Chem. Rev.,* 2004, vol. 248, 2425-2442
- **P.G. JESSOP.** Homogeneous Hydrogenation of Carbon Dioxide, in: The Handbook of Homogeneous Hydrogenation. Elsevier, 2007, vol. 1, 489-511
- Reactor Types and Their Industrial Applications. **K.D. HENKEL.** Ullmann's Encyclopedia of Industrial Chemistry. Wiley-VCH Verlag GmbH & Co. KGaA, 2005
- Liquid-Liquid Extraction. **E. MÜLLER et al.** Ullmann's Encyclopedia of Industrial Chemistry. Wiley-VCH Verlag GmbH & Co. KGaA, 2005
- **J.F. HARTWIG.** Organotransition Metal Chemistry. Universtiy Science Books, 2010, 545